# EUROPEAN PATENT APPLICATION

(11) **EP 4 671 373 A1**
(43) Date of publication of application: **31.12.2025**
(21) Application number: 24760340.0
(22) Date of filing: 20.02.2024
(51) Int. Cl.: C12N 15/55, C11D 7/42, C12N 1/15, C12N 1/19, C12N 1/21, C12N 5/10, C12N 9/20, C12N 15/63, C12Q 1/44

(54) **LIPASE VARIANT**

(30) Priority: 21.02.2023 JP 2023025140
(71) Applicant: Kao Corporation, Chuo-ku Tokyo 103-8210 (JP)
(72) Inventor: HIOKI, Takahiro, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2024/005941
(87) International publication number: WO 2024/177042

(57) **Abstract**

Provided is a lipase mutant with alleviated inhibition of activity against a dispersed substrate in the presence of a surfactant. The lipase mutant consists of an amino acid sequence having an identity of at least 75% with the amino acid sequence of SEQ ID NO: 2, 4, 6, or 8 and has a predetermined amino acid residue at a predetermined position numbered according to SEQ ID NO: 2.

## Description

### Field of the Invention

The present invention relates to a lipase mutant.

### Background of the Invention

Lipases are useful in various uses such as a laundry detergent, a dishwash detergent, oil processing, pulp treatment, feed, and pharmaceutical intermediate synthesis, and so on. In washing, lipases contribute to removal of stains including oil by hydrolyzing ester bonds in lipids to generate fatty acids.

As a lipase useful for washing, a *Thermomyces lanuginosus*-derived lipase (hereinafter, TLL) is commercially available under the trade name LIPOLASE (registered tradename). Patent Literature 1 discloses that *Cedecea* sp-16640 strain-derived lipase Lipr139 exhibits excellent cleansing performance compared to TLL. Patent Literature 2 discloses that a metagenome-derived lipase Lipr138 exhibits excellent cleansing performance compared to TLL.

As a method for sufficiently exhibiting the cleansing performance of a lipase, for example, Patent Literature 3 describes a method for washing oil stains by bringing a detergent including a lipase and a sulfosuccinate as a surfactant into contact with a hard article with oil stains and leaving it to stand without applying an external force. In an aqueous solution not containing a surfactant, very little triglycerides on a hard surface is removed by a lipase, but the removal of triglycerides on a hard surface by a lipase is notably enhanced in a washing solution containing a sulfosuccinate. However, even in the absence of a surfactant, lipases can efficiently decompose an ester substrate dispersed in an aqueous solution.

Patent Literature 1: JP-A-2015-523078
Patent Literature 2: JP-A-2015-525248
Patent Literature 3: JP-A-2021-17508

### Summary of the Invention

The present invention relates to the following 1) to 8).
1) A lipase mutant that consists of an amino acid sequence having an identity of at least 75% with the amino acid sequence of SEQ ID NO: 2, 4, 6, or 8 and has one or more amino acid residues selected from the group consisting of the following (a) to (e):
   (a) an amino acid residue other than leucine at a position corresponding to position 120 numbered according to SEQ ID NO: 2;
   (b) an amino acid residue other than serine at a position corresponding to position 130 numbered according to SEQ ID NO: 2;
   (c) an amino acid residue other than alanine at a position corresponding to position 134 numbered according to SEQ ID NO: 2;
   (d) an amino acid residue other than leucine at a position corresponding to position 136 numbered according to SEQ ID NO: 2; and
   (e) an amino acid residue other than serine at a position corresponding to position 137 numbered according to SEQ ID NO: 2,
   (provided that, when the lipase mutant has only an amino acid residue of (d) in the above (a) to (e) and the amino acid residue is methionine, the lipase mutant consists of an amino acid sequence having an identity of at least 93% with the amino acid sequence of SEQ ID NO: 2, 4, or 6).
2) A polynucleotide encoding the lipase mutant according to 1).
3) A vector or DNA fragment comprising the polynucleotide according to 2).
4) A transformed cell containing the vector or DNA fragment according to 3).
5) A cleansing composition containing the lipase mutant according to 1).
6) A stain-washing method comprising using the cleansing composition according to 5).
7) A method for producing a lipase mutant, comprising performing one or more steps selected from the group consisting of the following (i) to (v) in a polypeptide that consists of an amino acid sequence having an identity of at least 75% with the amino acid sequence of SEQ ID NO: 2, 4, 6, or 8 and has a lipase activity:
   (i) a step of substituting an amino acid residue at a position corresponding to position 120 numbered according to SEQ ID NO: 2 with an amino acid residue other than leucine;
   (ii) a step of substituting an amino acid residue at a position corresponding to position 130 numbered according to SEQ ID NO: 2 with an amino acid residue other than serine;
   (iii) a step of substituting an amino acid residue at a position corresponding to position 134 numbered according to SEQ ID NO: 2 with an amino acid residue other than alanine;
   (iv) a step of substituting an amino acid residue at a position corresponding to position 136 numbered according to SEQ ID NO: 2 with an amino acid residue other than leucine; and
   (v) a step of substituting an amino acid residue at a position corresponding to position 137 numbered according to SEQ ID NO: 2 with an amino acid residue other than serine,
   (provided that, when the method comprises performing only the step (iv) in the above (i) to (v) and the amino acid is substituted with methionine, the polypeptide consists of an amino acid sequence having an identity of at least 93% with the amino acid sequence of SEQ ID NO: 2, 4, or 6).
8) A method for evaluating or selecting a lipase having an ability to decompose an oil stain adhering to a hard surface in the presence of a sulfosuccinate or a salt thereof, comprising steps of:
   measuring lipase activity of a test lipase against a dispersed substrate in the presence of a surfactant;
   evaluating a degree of inhibition by the surfactant of lipase activity of the test lipase based on the measured result; and
   evaluating or selecting a test lipase which has a lower degree of lipase activity inhibition by the surfactant than a standard lipase as a lipase having an ability to decompose an oil stain adhering to a hard surface in the presence of a sulfosuccinate or a salt thereof.

### Brief Description of the Drawings

Figure 1 shows an enzymatic effect on detergency of each lipase in model washing solutions.
Figure 2 shows an enzymatic effect on detergency of each lipase in model washing solutions.
Figure 3 shows a correlation between the enzymatic effect on detergency of each lipase in a model washing solution and the degree of the alleviation of lipase activity inhibition.
Figure 4 shows the detergency of each lipase in model washing solutions.
Figure 5 shows an enzymatic effect on detergency of each lipase in model washing solutions.

### Detailed Description of the Invention

All Patent Literatures, Non Patent Literatures, and other publications cited herein are incorporated by reference in their entireties.

As used herein, the term "lipase" refers to triacyl glycerol lipase (EC3.1.1.3) and means an enzyme group that has an activity which hydrolyze an ester bond in a lipid to generate a fatty acid.

As used herein, the identity between amino acid sequences or nucleotide sequences is calculated by the Lipman-Pearson method (Science, 1985, 227: 1435-1441). Specifically, the identity is calculated by analysis using the homology analysis (Search homology) program of genetic information processing software GENETYX Ver. 12 by setting the Unit size to compare (ktup) to 2.

As used herein, the term "an identity of at least 75%" with respect to an amino acid sequence or nucleotide sequence refers to an identity of 75% or more, preferably 80% or more, more preferably 85% or more, further preferably 90% or more, further preferably 93% or more, further preferably 94% or more, further preferably 95% or more, further preferably 96% or more, further preferably 97% or more, further preferably 98% or more, further preferably 99% or more, and further preferably 99.5% or more.

As used herein, a "corresponding position" on an amino acid sequence or nucleotide sequence can be determined by aligning a target sequence and a reference sequence (for example, the amino acid sequence represented by SEQ ID NO: 2) so as to give the maximum homology. The alignment of amino acid sequences or nucleotide sequences can be carried out using a known algorithm, and the procedure thereof is known to those skilled in the art. For example, the alignment can be performed by using Clustal W multiple alignment program (Thompson, J.D., et al., 1994, Nucleic Acids Res., 22: 4673-4680) with the default set. Alternatively, Clustal W2 or Clustal omega, which are revised editions of Clustal W, can also be used. Clustal W, Clustal W2, and Clustal omega can be used on the website of, for example, the Clustal [www.clustal.org] operated by University College Dublin, the European Bioinformatics Institute: EBI [www.ebi.ac.uk/index.html], or the DNA data bank of Japan (DDBJ [www.ddbj.nig.ac.jp/searches-j.html]) operated by the National Institute of Genetics. A position in a target sequence aligned at any position in a reference sequence by the above-described alignment is considered to be a "position corresponding" to the position.

Those skilled in the art can further finely adjust and optimize the alignment of the amino acid sequence obtained above. The optimum alignment is preferably determined by considering the similarity of amino acid sequences, the frequency of gaps to be inserted, and so on. Here, the similarity of amino acid sequences refers to, when two amino acid sequences are aligned, the ratio (%) of the number of positions where the same or similar amino acid residues are present in both sequences to the number of the full-length amino acid residues. The similar amino acid residues mean that the amino acid residues have similar properties in the polarity or electric charge to each other and cause so-called conservative substitution among 20 amino acids constituting a protein. Groups consisting of such similar amino acid residues are well known to those skilled in the art, and examples thereof include, but not limited to, arginine and lysine or glutamine; glutamic acid and aspartic acid or glutamine; serine and threonine or alanine; glutamine and asparagine or arginine; and leucine and isoleucine.

As used herein, the term "amino acid residue" means 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

As used herein, an amino acid position and mutant are designated using the accepted IUPAC one-letter amino acid abbreviation as follows.

An amino acid at a predetermined position is designated by [amino acid, position]. For example, leucine at position 120 is shown as "L120".

"Substitution" of an amino acid is designated by [original amino acid, position, substituted amino acid]. For example, substitution of leucine at position 120 with alanine is designated as "L120A".

As used herein, the term "operable linkage" between a regulatory region, such as a promoter, and a gene indicates that a gene and a regulatory region are linked such that the gene can be expressed under the control of the regulatory region. The procedure of "operable linkage" between a gene and a regulatory region is known to those skilled in the art.

As used herein, the terms "upstream" and "downstream" with respect to a gene refer to the upstream and downstream of the gene in the transcription direction. For example, "a gene placed downstream of a promoter" means that the gene is present on the 3' side of the promoter in a DNA sense strand, and upstream of a gene means a region on the 5' side of the gene in a DNA sense strand.

As used herein, the "parent" polypeptide of a given mutant polypeptide refers to a polypeptide that brings about the mutant polypeptide by a predetermined mutation of an amino acid residue. In other words, the "parent" polypeptide is a polypeptide before the mutation is introduced into the mutant polypeptide. Such a parent polypeptide can be a natural (wild-type) polypeptide or its mutant.

The present inventors found an unexpected phenomenon that a washing solution containing a sulfosuccinate (hereinafter, washing solution) notably enhances the removal of triglycerides from hard surfaces by a lipase (Patent Literature 3) but highly inhibits the lipase activity against the ester substrate dispersed in the washing solution (hereinafter, dispersed substrate). The activity of Lipr139 and Lipr138 known to be suitable for washing to decompose the dispersed substrate in the presence of a sulfosuccinate was inhibited to 5% or less, and hardly detectable. Accordingly, a lipase exhibiting high detergency and decreased inhibition of its lipase activity against the dispersed substrate in the presence of a surfactant is desired.

The present inventors obtained lipase mutants in which the inhibition of the lipase activity against a dispersed substrate is alleviated in the presence of a surfactant, compared to the parent lipase. These lipase mutants also have notably improved ability to remove triglycerides on a hard surface in the presence of a surfactant. The present inventors also found that the degree of alleviation of the inhibition of lipase activity of a lipase against the dispersed substrate in the presence of a surfactant strongly correlates with the improvement of the ability to remove triglycerides on a hard surface in the presence of a sulfosuccinate and that, accordingly, a lipase having an ability to decompose oil stains adhering to hard surfaces in the presence of a sulfosuccinate can be evaluated or selected using the degree of inhibition of lipase activity against a dispersed substrate in the presence of a surfactant as an indicator.

The lipase mutant of the present invention has alleviated inhibition of lipase activity against a dispersed substrate in the presence of a surfactant, improved ability to remove triglycerides on a hard surface in the presence of a surfactant, and excellent detergency, compared to the parent lipase. According to the method for evaluating or selecting a lipase of the present invention, it is possible to simply and sensitively evaluate or select a lipase that exhibits high detergency against oil stains on hard surfaces in the presence of a sulfosuccinate or a salt thereof.

### <1. Lipase mutant and method for producing it>

The present invention provides a lipase mutant having excellent detergency and a method for producing it.

In one aspect, the present invention provides a method for producing a lipase mutant. The method of the present invention includes substitution of an amino acid residue at a predetermined position numbered according to SEQ ID NO: 2 with another amino acid residue in a parent lipase.

An example of the parent lipase of the lipase mutant of the present invention is a polypeptide that consists of an amino acid sequence having an identity of at least 75% with the amino acid sequence of SEQ ID NO: 2 and has lipase activity. Here, the polypeptide that consists of the amino acid sequence of SEQ ID NO: 2 and has lipase activity is lipase CnLip (NCBI Accession No. WP_061278013.1) derived from *Cedecea neteri.* The parent lipase that is a polypeptide that consists of an amino acid sequence having an identity of at least 75% with the amino acid sequence of SEQ ID NO: 2 and has lipase activity preferably has L at a position corresponding to position 120 numbered according to SEQ ID NO: 2, S at a position corresponding to position 130 numbered according to SEQ ID NO: 2, A at a position corresponding to position 134 numbered according to SEQ ID NO: 2, L at a position corresponding to position 136 numbered according to SEQ ID NO: 2, or S at a position corresponding to position 137 numbered according to SEQ ID NO: 2, and more preferably has L and S at positions corresponding to positions 120 and 130, respectively, numbered according to SEQ ID NO: 2, L and A at positions corresponding to positions 120 and 134, respectively, numbered according to SEQ ID NO: 2, L at positions corresponding to positions 120 and 136 numbered according to SEQ ID NO: 2, L and S at positions corresponding to positions 120 and 137, respectively, numbered according to SEQ ID NO: 2, S and A at positions corresponding to positions 130 and 134, respectively, numbered according to SEQ ID NO: 2, S and L at positions corresponding to positions 130 and 136, respectively, numbered according to SEQ ID NO: 2, S at positions corresponding to positions 130 and 137 numbered according to SEQ ID NO: 2, L and S at positions corresponding to positions 136 and 137, respectively, numbered according to SEQ ID NO: 2, L, S, and L at positions corresponding to positions 120, 130, and 136, respectively, numbered according to SEQ ID NO: 2, or L, S, L, and S at positions corresponding to positions 120, 130, 136, and 137, respectively, numbered according to SEQ ID NO: 2. In the amino acid sequence of SEQ ID NO: 2, positions corresponding to positions 120, 130, 134, 136, and 137 numbered according to SEQ ID NO: 2 are positions 120, 130, 134, 136, and 137, respectively.

Another example of the parent lipase of the lipase mutant of the present invention is a polypeptide that consists of an amino acid sequence having an identity of at least 75% with the amino acid sequence of SEQ ID NO: 4 and has lipase activity. Here, the polypeptide that consists of the amino acid sequence of SEQ ID NO: 4 and has lipase activity is a lipase EbLip (NCBI Accession No. MRT57156.1) derived from *Enterobacteriaceae* bacterium. The parent lipase that is a polypeptide consisting of an amino acid sequence having an identity of at least 75% with the amino acid sequence of SEQ ID NO: 4 and having lipase activity preferably has L at a position corresponding to position 120 numbered according to SEQ ID NO: 2, S at a position corresponding to position 130 numbered according to SEQ ID NO: 2, A at a position corresponding to position 134 numbered according to SEQ ID NO: 2, L at a position corresponding to position 136 numbered according to SEQ ID NO: 2, or S at a position corresponding to position 137 numbered according to SEQ ID NO: 2, and more preferably has L and S at positions corresponding to positions 120 and 130, respectively, numbered according to SEQ ID NO: 2, L and A at positions corresponding to positions 120 and 134, respectively, numbered according to SEQ ID NO: 2, L at positions corresponding to positions 120 and 136 numbered according to SEQ ID NO: 2, L and S at positions corresponding to positions 120 and 137, respectively, numbered according to SEQ ID NO: 2, S and A at positions corresponding to positions 130 and 134, respectively, numbered according to SEQ ID NO: 2, S and L at positions corresponding to positions 130 and 136, respectively, numbered according to SEQ ID NO: 2, S at positions corresponding to positions 130 and 137 numbered according to SEQ ID NO: 2, L and S at positions corresponding to positions 136 and 137, respectively, numbered according to SEQ ID NO: 2, or L, S, A, L, and S at positions corresponding to positions 120, 130, 134, 136, and 137, respectively, numbered according to SEQ ID NO: 2. In the amino acid sequence of SEQ ID NO: 4, positions corresponding to positions 120, 130, 134, 136, and 137 numbered according to SEQ ID NO: 2 are positions 120, 130, 134, 136, and 137, respectively.

Another example of the parent lipase of the lipase mutant of the present invention is a polypeptide that consists of an amino acid sequence having an identity of at least 75% with the amino acid sequence of SEQ ID NO: 6 and has lipase activity. Here, the polypeptide consisting of the amino acid sequence of SEQ ID NO: 6 and having lipase activity is lipase Ag1Lip derived from *Enterobacter* sp. (NCBI Accession No. EJF30243.1). The parent lipase that is a polypeptide consisting of an amino acid sequence having an identity of at least 75% with the amino acid sequence of SEQ ID NO: 6 and having lipase activity preferably has L at a position corresponding to position 120 numbered according to SEQ ID NO: 2, S at a position corresponding to position 130 numbered according to SEQ ID NO: 2, A at a position corresponding to position 134 numbered according to SEQ ID NO: 2, L at a position corresponding to position 136 numbered according to SEQ ID NO: 2, or S at a position corresponding to position 137 numbered according to SEQ ID NO: 2, and more preferably has L and S at positions corresponding to positions 120 and 130, respectively, numbered according to SEQ ID NO: 2, L and A at positions corresponding to positions 120 and 134, respectively, numbered according to SEQ ID NO: 2, L at positions corresponding to positions 120 and 136 numbered according to SEQ ID NO: 2, L and S at positions corresponding to positions 120 and 137, respectively, numbered according to SEQ ID NO: 2, S and A at positions corresponding to positions 130 and 134, respectively, numbered according to SEQ ID NO: 2, S and L at positions corresponding to positions 130 and 136, respectively, numbered according to SEQ ID NO: 2, S at positions corresponding to positions 130 and 137 numbered according to SEQ ID NO: 2, or L and S at positions corresponding to positions 136 and 137, respectively, numbered according to SEQ ID NO: 2. In the amino acid sequence of SEQ ID NO: 6, positions corresponding to positions 120, 130, 134, 136, and 137 numbered according to SEQ ID NO: 2 are positions 120, 130, 134, 136, and 137, respectively.

Another example of the parent lipase of the lipase mutant of the present invention is a polypeptide that consists of an amino acid sequence having an identity of at least 75% with the amino acid sequence of SEQ ID NO: 8 and has lipase activity. Here, the polypeptide that consists of the amino acid sequence of SEQ ID NO: 8 and has lipase activity is lipase CspLip (NCBI Accession No. WP_016537805.1) derived from *Cedecea* sp. The parent lipase that is a polypeptide consisting of an amino acid sequence having an identity of at least 75% with the amino acid sequence of SEQ ID NO: 8 and having lipase activity preferably has L at a position corresponding to position 120 numbered according to SEQ ID NO: 2, S at a position corresponding to position 130 numbered according to SEQ ID NO: 2, A at a position corresponding to position 134 numbered according to SEQ ID NO: 2, M at a position corresponding to position 136 numbered according to SEQ ID NO: 2, or S at a position corresponding to position 137 numbered according to SEQ ID NO: 2, and more preferably has L and S at positions corresponding to positions 120 and 130, respectively, numbered according to SEQ ID NO: 2, L and A at positions corresponding to positions 120 and 134, respectively, numbered according to SEQ ID NO: 2, L and M at positions corresponding to positions 120 and 136, respectively, numbered according to SEQ ID NO: 2, L and S at positions corresponding to positions 120 and 137, respectively, numbered according to SEQ ID NO: 2, S and A at positions corresponding to positions 130 and 134, respectively, numbered according to SEQ ID NO: 2, S and M at positions corresponding to positions 130 and 136, respectively, numbered according to SEQ ID NO: 2, S at positions corresponding to positions 130 and 137 numbered according to SEQ ID NO: 2, or M and S at positions corresponding to positions 136 and 137, respectively, numbered according to SEQ ID NO: 2. In the amino acid sequence of SEQ ID NO: 8, positions corresponding to positions 120, 130, 134, 136, and 137 numbered according to SEQ ID NO: 2 are positions 121, 131, 135, 137, and 138, respectively.

When the lipase mutant of the present invention is produced from a parent lipase, one or more, preferably two or more, more preferably three or more, further preferably four or more, and further preferably all steps selected from the group consisting of the following (i) to (v) are performed for the parent lipase:
(i) a step of substituting the amino acid residue at a position corresponding to position 120 numbered according to SEQ ID NO: 2 with an amino acid residue other than L;
(ii) a step of substituting the amino acid residue at a position corresponding to position 130 numbered according to SEQ ID NO: 2 with an amino acid residue other than S;
(iii) a step of substituting the amino acid residue at a position corresponding to position 134 numbered according to SEQ ID NO: 2 with an amino acid residue other than A;
(iv) a step of substituting the amino acid residue at a position corresponding to position 136 numbered according to SEQ ID NO: 2 with an amino acid residue other than L; and
(v) a step of substituting the amino acid residue at a position corresponding to position 137 numbered according to SEQ ID NO: 2 with an amino acid residue other than S, (provided that, when the method includes performing only the step (iv) in the above (i) to (v) and the amino acid residue is substituted with M, the parent lipase consists of an amino acid sequence having an identity of at least 93% with the amino acid sequence of SEQ ID NO: 2, 4, or 6).

The above (i) to (v) are further preferably the following (i') to (v'), respectively:
(i') a step of substituting the amino acid residue at a position corresponding to position 120 numbered according to SEQ ID NO: 2 with A, F, G, H, I, K, M, N, Q, R, S, T, V, W, or Y;
(ii') a step of substituting the amino acid residue at a position corresponding to position 130 numbered according to SEQ ID NO: 2 with A, C, E, F, G, H, I, K, L, M, Q, R, T, V, W, or Y;
(iii') a step of substituting the amino acid residue at a position corresponding to position 134 numbered according to SEQ ID NO: 2 with C, D, E, G, I, T, or V;
(iv') a step of substituting the amino acid residue at a position corresponding to position 136 numbered according to SEQ ID NO: 2 with A, C, E, F, G, H, I, M, S, T, V, or W; and
(v') a step of substituting the amino acid residue at a position corresponding to position 137 numbered according to SEQ ID NO: 2 with C, N, or T,
(provided that, when the method includes performing only the step (iv') in the above (i') to (v') and the amino acid residue is substituted with M, the parent lipase consists of an amino acid sequence having an identity of at least 93% with the amino acid sequence of SEQ ID NO: 2, 4, or 6).

The present invention also provides a lipase mutant. The lipase mutant of the present invention is a polypeptide that consists of an amino acid sequence obtained by substituting an amino acid residue at a predetermined position numbered according to SEQ ID NO: 2 in the amino acid sequence of the parent lipase with another amino acid residue and has lipase activity.

The lipase mutant of the present invention consists of an amino acid sequence that has an identity of at least 75% with the amino acid sequence of SEQ ID NO: 2, 4, 6, or 8 and has one or more, preferably two or more, more preferably three or more, further preferably four or more, and further preferably all amino acid residues selected from the group consisting of the following (a) to (e):
(a) an amino acid residue other than L at a position corresponding to position 120 numbered according to SEQ ID NO: 2;
(b) an amino acid residue other than S at a position corresponding to position 130 numbered according to SEQ ID NO: 2;
(c) an amino acid residue other than A at a position corresponding to position 134 numbered according to SEQ ID NO: 2;
(d) an amino acid residue other than L at a position corresponding to position 136 numbered according to SEQ ID NO: 2; and
(e) an amino acid residue other than S at a position corresponding to position 137 numbered according to SEQ ID NO: 2,
(provided that, when the lipase mutant has only an amino acid residue of (d) in the above (a) to (e) and the amino acid residue is M, the lipase mutant consists of an amino acid sequence having an identity of at least 93% with the amino acid sequence of SEQ ID NO: 2, 4, or 6).

The above (a) to (e) are further preferably the following (a') to (e'), respectively:
(a') A, F, G, H, I, K, M, N, Q, R, S, T, V, W, or Y at a position corresponding to position 120 numbered according to SEQ ID NO: 2;
(b') A, C, E, F, G, H, I, K, L, M, Q, R, T, V, W, or Y at a position corresponding to position 130 numbered according to SEQ ID NO: 2;
(c') C, D, E, G, I, T, or V at a position corresponding to position 134 numbered according to SEQ ID NO: 2;
(d') A, C, E, F, G, H, I, M, S, T, V, or W at a position corresponding to position 136 numbered according to SEQ ID NO: 2; and
(e') C, N, or T at a position corresponding to position 137 numbered according to SEQ ID NO: 2,
(provided that, when the lipase mutant has only M of (d') in the above (a') to (e'), the lipase mutant consists of an amino acid sequence having an identity of at least 93% with the amino acid sequence of SEQ ID NO: 2, 4, or 6).

In a preferable embodiment, the lipase mutant of the present invention is a lipase mutant that consists of an amino acid sequence having an identity of at least 75% with the amino acid sequence of SEQ ID NO: 2, 4, 6, or 8 and has any of amino acid residues Nos. 1 to 75 in Table 1 below. In these lipase mutants, more preferred are a lipase mutant that consists of an amino acid sequence having an identity of at least 75% with the amino acid sequence of SEQ ID NO: 2 and has any of amino acid residues Nos. 1 to 74 in Table 1, a lipase mutant that consists of an amino acid sequence having an identity of at least 75% with the amino acid sequence of SEQ ID NO: 4 and has any of amino acid residues Nos. 11, 59, and 75 in Table 1, a lipase mutant that consists of an amino acid sequence having an identity of at least 75% with the amino acid sequence of SEQ ID NO: 6 and has the amino acid residue No. 59 in Table 1, and a lipase mutant that consists of an amino acid sequence having an identity of at least 75% with the amino acid sequence of SEQ ID NO: 8 and has any of amino acid residues Nos. 4, 6, 11, 22, 24, 30, 36, 41, 43, 50, 51, and 59 in Table 1.

**[Table 1]**

| No. | Amino acid residue |
|---|---|
| 1 | 120A |
| 2 | 120F |
| 3 | 120G |
| 4 | 120H |
| 5 | 120I |
| 6 | 120K |
| 7 | 120M |
| 8 | 120N |
| 9 | 120Q |
| 10 | 120R |
| 11 | 120S |
| 12 | 120T |
| 13 | 120V |
| 14 | 120W |
| 15 | 120Y |
| 16 | 130A |
| 17 | 130C |
| 18 | 130E |
| 19 | 130F |
| 20 | 130H |
| 21 | 130I |
| 22 | 130K |
| 23 | 130L |
| 24 | 130M |
| 25 | 130Q |
| 26 | 130R |
| 27 | 130T |
| 28 | 130V |
| 29 | 130W |
| 30 | 130Y |
| 31 | 134C |
| 32 | 134D |
| 33 | 134E |
| 34 | 134G |
| 35 | 134I |
| 36 | 134T |
| 37 | 134V |
| 38 | 136A |
| 39 | 136C |
| 40 | 136E |
| 41 | 136F |
| 42 | 136G |
| 43 | 136H |
| 44 | 136I |
| 45 | 136M |
| 46 | 136T |
| 47 | 136V |
| 48 | 136W |
| 49 | 137C |
| 50 | 137N |
| 51 | 137T |
| 52 | 120G 130A |
| 53 | 120G 130L |
| 54 | 120H 130A |
| 55 | 120H 130L |
| 56 | 120R 130A |
| 57 | 120R 130K |
| 58 | 120R 130L |
| 59 | 120S 130A |
| 60 | 120S 130K |
| 61 | 120S 130L |
| 62 | 120S 134T |
| 63 | 120S 136A |
| 64 | 120S 136S |
| 65 | 120S 137N |
| 66 | 130A 134T |
| 67 | 130A 136M |
| 68 | 130A 137T |
| 69 | 130K 136M |
| 70 | 130M 137T |
| 71 | 130Y 137T |
| 72 | 136M 137N |
| 73 | 120S 130A 136M |
| 74 | 120R 130K 136M 137N |
| 75 | 120R 130A 134T 135V 136M 137N |

| | |
|---|---|
| *The amino acid position is a position numbered according to SEQ ID NO: 2. | |

The substitution of an amino acid residue at the above predetermined position is substitution for alleviating the inhibition of lipase activity against a dispersed substrate in the presence of a surfactant and is substitution for improving the ability of lipase to remove triglycerides on a hard surface in the presence of a surfactant. In other words, the substitution of an amino acid residue at the above predetermined position is substitution for improving the detergency of the lipase. Accordingly, the lipase mutant of the present invention has improved detergency compared to a parent lipase, i.e., a lipase before substitution of an amino acid residue at a predetermined position. All of positions 120, 130, 134, 136, and 137 numbered according to SEQ ID NO: 2 are present in a lid domain (the lid of the active center located at positions from 108 to 161 numbered according to SEQ ID NO: 2) of the lipase.

The lipase mutant of the present invention may have a mutation (for example, deletion, substitution, addition, or insertion) at any position of the parent lipase, in addition to the mutations at the above predetermined positions, as long as the detergency is not prevented. The mutation may be naturally generated or may be artificially introduced.

### <2. Polynucleotide encoding lipase mutant of the present invention>

The lipase mutant of the present invention can be produced using various mutagenesis techniques known in the art. For example, the lipase mutant can be produced by mutating a polynucleotide encoding the amino acid residue to be substituted in a parent lipase gene (reference lipase gene) encoding the reference amino acid sequence into a polynucleotide encoding an amino acid residue after substitution, and further expressing the mutant from the mutant gene.

The polynucleotide encoding the lipase mutant of the present invention can be in the form of single-stranded or double-stranded DNA, RNA, or artificial nucleic acid or can be cDNA or chemically synthesized DNA containing no introns.

In the present invention, as the method for mutating an amino acid residue of a parent lipase, various mutagenesis techniques known in the art can be used. For example, a polynucleotide encoding the lipase mutant of the present invention can be obtained by mutating a nucleotide sequence encoding an amino acid residue to be mutated into a nucleotide sequence encoding the amino acid residue after mutation in a polynucleotide encoding the amino acid sequence of a parent lipase (hereinafter, also referred to as parent gene).

Basically, introduction of a desired mutation into a parent gene can be performed using various site-directed mutagenesis methods known to those skilled in the art. The site-directed mutagenesis can be performed by an arbitrary method, such as an inverse PCR method or an annealing method. Commercially available site-directed mutagenesis kits (for example, QuickChange II Site-Directed Mutagenesis Kit and QuickChange Multi Site-Directed Mutagenesis Kit of Stratagene) can also be used.

Site-directed mutagenesis into a parent gene can be most generally performed using a mutation primer including a nucleotide mutation to be introduced. The mutation primer may be designed so as to anneal with a region including a nucleotide sequence that encodes an amino acid residue to be mutated in a parent gene and to include a nucleotide sequence having a nucleotide sequence (codon) that encodes an amino acid residue after the mutation instead of the nucleotide sequence (codon) encoding the amino acid residue to be mutated. Nucleotide sequences (codons) encoding amino acid residues before and after mutation can be appropriately recognized and selected by those skilled in the art based on ordinary textbooks and the like. Alternatively, as site-directed mutagenesis, a method of linking DNA fragments into one by SOE (splicing by overlap extension)-PCR (Gene, 1989, 77(1): 61-68), wherein the DNA fragments are separately obtained by amplifying both the upstream and downstream sides of a mutation site with two complementary primers including the nucleotide mutation to be introduced can be used.

A template DNA including a parent gene can be prepared from a microorganism that produces the above-described parent lipase by extracting genomic DNA by a usual method or extracting RNA and synthesizing cDNA therefrom by reverse transcription. Alternatively, a corresponding nucleotide sequence chemically synthesized based on the amino acid sequence of a parent lipase may be used as a template DNA. DNA sequences including nucleotide sequences encoding lipases consisting of amino acid sequences of SEQ ID NOs: 2, 4, 6, and 8 are represented by SEQ ID NOs: 1, 3, 5, and 7, respectively.

A mutation primer can be produced by a known oligonucleotide synthesis method such as a phosphoramidite method (Nucleic Acids Research, 1989, 17: 7059-7071). Such primer synthesis can also be carried out using, for example, a commercially available oligonucleotide synthesis apparatus (for example, manufactured by ABI Inc.). A polynucleotide encoding a lipase mutant with a desired mutation of the present invention can be obtained using a primer set including the mutation primer and performing site-directed mutagenesis as described above using a parent gene as a template DNA.

The polynucleotide encoding the lipase mutant of the present invention can include a single-stranded or double-stranded DNA, cDNA, RNA, or another artificial nucleic acid. The DNA, cDNA, and RNA may be chemically synthesized. The polynucleotide may include a nucleotide sequence of an untranslated region (UTR), in addition to an open reading frame (ORF). The polynucleotide may be codon-optimized in accordance with the type of a transformant for producing the mutant polypeptide of the present invention. Information on codons to be used by various organisms is available from the Codon Usage Database ([www.kazusa.or.jp/codon/]).

### <3. Vector or DNA fragment>

The obtained polynucleotide encoding the lipase mutant of the present invention can be incorporated into a vector. The type of the vector containing the polynucleotide is not particularly limited and may be any vector such as a plasmid, phage, phagemid, cosmid, virus, YAC vector, and shuttle vector. The vector is, but not limited to, preferably, a vector capable of being amplified in bacteria, preferably, in *Bacillus* bacteria (for example, *Bacillus subtilis* or its mutant strain), more preferably, an expression vector capable of inducing expression of a transgene in *Bacillus* bacteria. In particular, a shuttle vector, which is a vector capable of replicating in both *Bacillus* bacteria and other organisms, can be suitably used for recombinant production of the lipase mutant of the present invention. Preferable examples of the vector include, but not limited to, shuttle vectors, such as pHA3040SP64, pHSP64R, and pASP64 (Japanese Patent No. 3492935), pHY300PLK (expression vector capable of transforming both *Escherichia coli* and *Bacillus subtilis;* Jpn J. Genet., 1985, 60: 235-243), and pAC3 (Nucleic Acids Res., 1988, 16: 8732); and plasmid vectors that can be used for transformation of *Bacillus* bacteria, such as pUB110 (J. Bacteriol., 1978, 134: 318-329) and pTA10607 (Plasmid, 1987, 18: 8-15). In addition, plasmid vectors derived from *Escherichia coli* (e.g., pET22b(+), pBR322, pBR325, pUC57, pUC118, pUC119, pUC18, pUC19, and pBluescript) can also be used.

The above vectors can include a DNA replication initiation region or a DNA region including replication origin. Alternatively, in the above vectors, a control sequence may be operably linked to the upstream of a polynucleotide encoding the lipase mutant of the present invention (i.e., lipase mutant gene), wherein the control sequence is a promoter region for initiating transcription of the gene, terminator region, secretory signal region for extracellularly secreting the expressed protein, or the like.

The type of the control sequence, such as the promoter region, terminator region, and secretory signal region, is not particularly limited, and a promoter or secretory signal sequence that is usually used can be appropriately selected and used depending on the host into which the sequence is introduced. For example, suitable examples of the control sequence capable of being incorporated into a vector include a promoter of the cellulase gene of *Bacllus* sp. KSM-S237 strain and a secretary signal sequence.

Alternatively, a marker gene (e.g., a gene resistant to drugs, such as ampicillin, neomycin, kanamycin, and chloramphenicol) for selecting a host into which the vector has been appropriately introduced may be further incorporated into the vector of the present invention. Alternatively, when an auxotrophic strain is used as the host, a gene encoding an enzyme that synthesizes a required nutrient may be incorporated into a vector as a marker gene. Alternatively, when a selection medium that requires a specific metabolism for growth is used, a gene related to the metabolism may be incorporated into a vector as a marker gene. Examples of such a metabolism-related gene include an acetamidase gene for using acetamide as a nitrogen source.

The polypeptide encoding the lipase mutant of the present invention may be linked to a control sequence and a marker gene by a method known in the art such as an SOE (splicing by overlap extension)-PCR method (Gene, 1989, 77: 61-68). The procedure of introducing the linked fragments into a vector is well known in the art.

### <4. Transformed cell>

A transformed cell of the present invention can be obtained by introducing a vector including a polynucleotide encoding the lipase mutant of the present invention into a host or by introducing a DNA fragment including a polynucleotide encoding the lipase mutant of the present invention into the genome of a host.

Examples of the host cell include microorganisms such as bacteria and fungi. Examples of the bacteria include *Escherichia coli* and bacteria belonging to *Staphylococcus, Enterococcus, Listeria,* and *Bacillus,* and among them, preferred are *Escherichia coli* and *Bacillus* bacteria, more preferred are *Bacillus* bacteria, and further preferred are *Bacillus subtilis* (e.g., *Bacillus subtilis* Marburg No. 168 strain and its mutant strain). Examples of the *Bacillus subtilis* mutant strain include a nine protease-deficient strain KA8AX described in J. Biosci. Bioeng., 2007, 104(2): 135-143 and D8PA strain that is an eight protease-deficient strain with improved protein folding efficiency described in Biotechnol. Lett., 2011, 33(9): 1847-1852. Examples of the fungus include *Trichoderma, Aspergillus,* and *Rhizopus.*

As the method for introducing a vector into a host, a method that usually used in the art, such as a protoplast method and an electroporation method, can be used. A desired transformant into which a vector has been introduced can be obtained by selecting a strain in which the introduction has been appropriately performed by using expression of a marker gene, auxotrophy, or the like as an indicator.

Alternatively, a fragment in which a polypeptide encoding the lipase mutant of the present invention, a control sequence, and a marker gene are linked can also be directly introduced into the genome of a host. For example, a DNA fragment in which a sequence complementary to the genome of a host has been added to both ends of the linked fragment is constructed by an SOE-PCR method or the like and is introduced into a host to cause homologous recombination between the host genome and the DNA fragment. Consequently, the polynucleotide encoding the lipase mutant of the present invention is introduced into the genome of the host.

When the thus-obtained transformant into which a polynucleotide encoding the lipase mutant of the present invention or a vector including it has been introduced is cultured in an appropriate medium, a gene encoding the protein on the vector is expressed to generate the lipase mutant of the present invention. The medium that is used for culturing the transformant can be appropriately selected depending on the type of the microorganism of the transformant by those skilled in the art.

Alternatively, the lipase mutant of the present invention may be expressed from a polynucleotide encoding the lipase mutant of the present invention or its transcriptional product using a cell-free translation system. The "cell-free translation system" is an in vitro transcription translation system or in vitro translation system constructed by adding a reagent, such as amino acid, necessary for translation of a protein to a suspension obtained by mechanically lysing the cells as the host.

The lipase mutant of the present invention generated by the culture or cell-free translation system can be isolated or purified by using general methods that are used in protein purification, such as centrifugation, ammonium sulfate precipitation, gel chromatography, ion exchange chromatography, and affinity chromatography, alone or in appropriate combination. The protein collected from the culture may be further purified by a known method.

### <5. Cleansing composition>

The thus-obtained lipase mutant of the present invention has notably alleviated inhibition of lipase activity against a dispersed substrate in the presence of a surfactant, compared to the parent lipase. The lipase mutant of the present invention is notably improved in the ability to remove triglycerides on a hard surface in the presence of a surfactant, compared to the parent lipase. Since the lipase mutant of the present invention can remove triglycerides on a hard surface in the presence of a surfactant even without applying an external force, it is possible to remove triglycerides not only on a hard surface but also on a soft surface. Accordingly, the lipase mutant of the present invention is excellent in detergency and exhibits good detergency even in the presence of a surfactant. Here, the term "detergency" means an ability to provide removal of stains, especially oil stains in a laundering or washing process.

Examples of the surfactant include one of an anionic surfactant, a nonionic surfactant, an amphoteric surfactant, and a cationic surfactant or a combination thereof and preferably one of an anionic surfactant and a nonionic surfactant or a combination thereof, more preferably one of a sulfosuccinate or its salt, SDS, and Triton X-100 or a combination thereof described in detail below, and further preferably a sulfosuccinate or its salt.

The "dispersed substrate" refers to an ester substrate that includes an ester bond susceptible to hydrolysis by a lipase and is solubilized, emulsified, or dispersed in an aqueous solution. Examples of the dispersed substrate include, for example, 4-nitrophenol fatty acid ester, triglyceride, methylresorufin ester, arachidonic acid-1-thioglycerol, fluorescein diacetate, fluorescein isothiocyanate diacetate, and EnzChek^{™} Lipase Substrate (Invitrogen^{™}) can be used. When the substrate is insoluble, it can also be used after emulsification by adding an emulsion stabilizer or an emulsifier. Among these substrates, from the viewpoint of convenience of lipase activity measurement, 4-nitrophenol fatty acid ester is preferable, 4-nitrophenol fatty acid ester with a fatty acid chain length of from 2 to 16 is more preferable, and 4-nitrophenyl octanoate is further preferable.

The "inhibition of lipase activity against a dispersed substrate in the presence of a surfactant" means that the lipase activity against a dispersed substrate in the presence of a surfactant is lower than the lipase activity against a dispersed substrate in the absence of a surfactant. The degree of inhibition of lipase activity against a dispersed substrate in the presence of a surfactant can be measured using a well-known method in the art. For example, lipase activity against a dispersed substrate in the presence of a surfactant is determined by mixing a lipase and a surfactant solution, adding a 4-nitrophenyl octanoate solution as a dispersed substrate to the resulting mixture solution, measuring the absorbance change (OD/min) at 405 nm due to release of 4-nitrophenol, and determining the difference ΔOD/min (lipase activity value in the surfactant solution) from a blank (sample without addition of a lipase). Separately, the ΔOD/min (lipase activity value in a buffer) when the buffer is used instead of the surfactant solution is determined. Subsequently, the ΔOD/min ratio is determined by dividing the lipase activity value in the surfactant solution by the lipase activity value in the buffer. The ΔOD/min ratio is a relative measure of the lipase activity retention rate in a surfactant solution compared to in a buffer, and a smaller value thereof shows that the lipase activity is inhibited in the presence of a surfactant. Accordingly, using such a value as an indicator, the degree of inhibition of lipase activity against a dispersed substrate in the presence of a surfactant can be evaluated. In addition, the degree of the alleviation of inhibition of a lipase activity, which shows how much the degree of inhibition of the lipase activity of a lipase mutant in the presence of a surfactant is alleviated compared to a parent lipase, can be determined by dividing the ΔOD/min ratio of the lipase mutant by the ΔOD/min ratio of the parent lipase. When the degree of the alleviation is larger than 1, the inhibition of lipase activity of the lipase mutant in the presence of a surfactant is alleviated compared to the parent lipase.

In the lipase mutant of the present invention, the degree of the alleviation ((ΔOD/min ratio of lipase mutant)/(ΔOD/min ratio of parent lipase)) under the conditions of (3) of Examples described later is preferably 1.1 or more, more preferably 1.2 or more, further preferably 1.3 or more, and further preferably 3.5 or more.

The term "hard surface" refers to a hard solid surface of an inanimate object. Examples of the inanimate object having such a hard surface include tableware, kitchen items, and/or items in the living environment such as a bathroom, a toilet area, and a floor. Specifically, examples of the tableware include materials and equipment that come into contact with food, for example, so-called tableware such as plates and bowls; storage containers such as Tupperware and bottles; cooking utensils such as knives, cutting boards, pots, frying pans, and fish grills; and cooking appliances such as food processors and mixers. The kitchen items are items that are used around the kitchen, and examples thereof include storage spaces for food, dishes, and cookware, such as a refrigerator and a cupboard; food cooking areas such as a drain, a kitchen table, a range hood, a sink, a gas range, and a microwave range; and floors and walls in the periphery of the storage spaces and cooking areas. The lipase mutant of the present invention is preferably used for removing triglycerides on hard surfaces of items selected from tableware, storage containers, cooking utensils, and cooking appliances.

The term "soft surface" refers to a soft solid surface of an inanimate object. Examples of the inanimate object having such a soft surface include fiber products. Here, the fiber product encompasses cloth fabric that has been made by weaving or knitting a large number of natural, regenerated, or synthetic fibers into thin, wide sheets and fabric products that have been made by sewing, pressing, and so on the resulting cloth fabric. Examples of the fabric product include clothing (e.g., underwear, shirts, sweaters, skirts, and sweatshirts), towels, accessories (e.g., slippers, scarves, stoles, gloves, and socks), equipment (e.g., supporters, masks, gauze, and bandages), bedding (e.g., duvet, mats, cushions, pillows, and blankets), covers (e.g., duvet covers, cushion covers, pillow covers, sheets, floor cushion covers, and toilet seat covers), and toys (e.g., stuffed toys).

The "triglyceride" includes an ester bond and can be hydrolyzed by a lipase. The triglyceride is not specifically limited but is preferably a triglyceride adhering or likely to be adhering to a hard surface, and examples thereof include triglycerides with fatty acids selected from the group consisting of palmitic acid, stearic acid, and oleic acid as constituent fatty acids. Examples of the oil including triglycerides include oils derived from animals such as cows and pigs and oils derived from plants, such as rapeseed oil and olive oil.

The "ability to remove triglycerides on a hard surface" of a lipase means an ability of the lipase to hydrolyze triglycerides adhering to a hard surface to thereby remove the triglycerides from the hard surface and can be an indicator of detergency. The ability to remove triglycerides on a hard surface in the presence of a surfactant can be evaluated using a well-known method in the art. For example, a triglyceride-containing model oil stain including a predetermined indicator substance (for example, a dye highly soluble in fat, such as Sudan III) is adhered to a hard surface, and a washing solution including a lipase and a surfactant is added thereto to perform washing treatment under predetermined conditions. A part of the washing solution is fractionated, the concentration of the indicator substance in the model oil stain solubilized in the washing solution by the washing treatment is measured by, for example, absorbance measurement, and the difference from before the washing can be determined as an indicator of the ability to remove triglycerides on a hard surface in the presence of a surfactant.

The lipase mutant of the present invention is useful as an enzyme that is blended in various cleansing compositions and is particularly useful as an enzyme that is blended in a cleansing composition suitable for low-temperature washing.

Here, the "low temperature" is, for example, 40°C or less, 35°C or less, 30°C or less, or 25°C or less and is, for example, 5°C or more, 10°C or more, or 15°C or more and is, for example, from 5°C to 40°C, from 10°C to 35°C, from 15°C to 30°C, or from 15°C to 25°C.

The amount of the lipase mutant of the present invention blended in the cleansing composition is not specifically limited as long as the lipase mutant exhibits the activity and is, based on 1 kg of the cleansing composition, for example, preferably 0.1 mg or more, more preferably 1 mg or more, and further preferably 5 mg or more and is preferably 5000 mg or less, further preferably 1 000 mg or less, and further preferably 500 mg or less, and is preferably from 0.1 to 5 000 mg, more preferably from 1 to 1 000 mg, and further preferably from 5 to 500 mg.

The cleansing composition preferably includes a sulfosuccinate or a salt thereof, in addition to the lipase mutant of the present invention. The sulfosuccinate or a salt thereof is known as a component that is blended in cleansing compositions (for example, JP-A-2019-182911). The sulfosuccinate or a salt thereof is preferably a branched alkyl sulfosuccinate having a branched-chain alkyl group having 9 or more and 12 or less carbon atoms or a salt thereof, more preferably a branched alkyl sulfosuccinate having a branched-chain alkyl group having 9 or 10 carbon atoms or a salt thereof, and further preferably a branched alkyl sulfosuccinate having a branched-chain alkyl group having 10 carbon atoms or a salt thereof. The sulfosuccinate or a salt thereof is a branched dialkyl sulfosuccinate or a salt thereof and is preferably a branched dialkyl sulfosuccinate in which two branched-chain alkyl groups are each a branched-chain alkyl group having 9 or more and 12 or less carbon atoms or a salt thereof, more preferably branched dialkyl sulfosuccinate in which two branched-chain alkyl groups are each a branched-chain alkyl group having 9 or 10 carbon atoms or a salt thereof, further preferably a branched dialkyl sulfosuccinate in which two branched-chain alkyl groups are each a branched-chain alkyl group having 10 carbon atoms or a salt thereof, and further preferably bis-(2-propylheptyl) sulfosuccinate or a salt thereof.

Examples of the salt include an alkali metal salt and an alkanolamine salt, and preferred is an alkali metal salt or an alkanolamine salt, more preferred is a salt selected from the group consisting of a sodium salt, a potassium salt, a triethanolamine salt, a diethanolamine salt, and a monoethanolamine salt, and further preferred is a sodium salt.

Examples of the sulfosuccinate or a salt thereof include compounds of the following Formula 1: 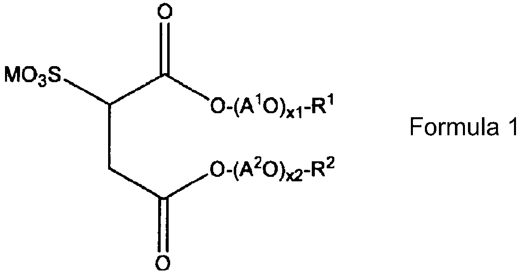 [in Formula 1, R¹ and R² are each a branched-chain alkyl group having 9 or more and 12 or less carbon atoms; A¹O and A²O are each an alkyleneoxy group having 2 or more and 4 or less carbon atoms; x1 and x2 are average addition mole numbers and are each a number of 0 or more and 10 or less; and M is a cation].

In Formula 1, R¹ are R² are each preferably a branched-chain alkyl group selected from the group consisting of a branched-chain nonyl group, a branched-chain decyl group, and a branched-chain dodecyl group and more preferably a branched-chain decyl group. The branched-chain decyl group is preferably a 2-propylheptyl group.

In Formula 1, A¹O and A²O are each an alkyleneoxy group having 2 or more and 4 or less carbon atoms and, preferably 2 or 3 carbon atoms from the viewpoint of lubricity to water. In Formula 1, x1 and x2 represent the average addition mole numbers of A¹O and A²O, respectively, and are each a number of 0 or more and 10 or less and, preferably 6 or less, more preferably 4 or less, and further preferably 2 or less, and are further preferably 0 from the viewpoint of lubricity to water.

In Formula 1, M is a cation. M is preferably a cation other than a hydrogen ion. Examples of M include an alkali metal ion such as a lithium ion, a sodium ion, and a potassium ion; an alkaline earth metal ion such as a calcium ion and a barium ion; and an organic ammonium ion such as a triethanolammonium ion, a diethanolammonium ion, a monoethanolammonium ion, a trimethylammonium ion, and a monomethylammonium ion.

M is preferably an alkali metal ion or an alkanolammonium ion, more preferably a sodium ion, a potassium ion, a triethanolammonium ion, a diethanolammonium ion, or a monoethanolammonium ion, and further preferably a sodium ion from the viewpoint of dispersibility in water.

The sulfosuccinate or a salt thereof is preferably a compound of the following Formula 1-1. The compound of Formula 1-1 is a compound with x1 and x2 in Formula 1 being each 0. 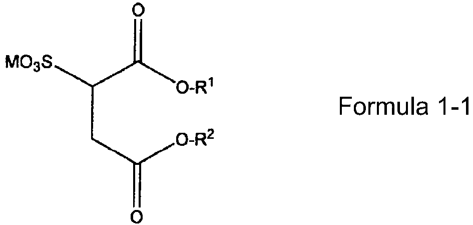 [in Formula 1-1, R¹ and R² are each a branched-chain alkyl group having 9 or more and 12 or less carbon atoms; and M is a cation].

Specific examples and preferable examples of R¹, R², and M in Formula 1-1 are the same as those in Formula 1.

In one preferable embodiment, the sulfosuccinate or a salt thereof is bis-(2-propylheptyl) sulfosuccinate or a salt thereof.

The amount of the sulfosuccinate or a salt thereof blended in the cleansing composition is preferably 0.01 mass% or more and more preferably 0.1 mass% or more and is preferably 30.0 mass% or less, more preferably 10.0 mass% or less, and further preferably 2.0 mass% or less. The amount is preferably from 0.01 to 30.0 mass%, more preferably from 0.1 to 10.0 mass%, and further preferably from 0.1 to 2.0 mass%.

The cleansing composition can include various enzymes in addition to the lipase mutant of the present invention, such as a hydrolase, an oxidase, a reductase, a transferase, a lyase, an isomerase, a ligase, and a synthetase. Among these enzymes, preferred are a lipase different from the lipase mutant of the present invention, an amylase, a protease, a cellulase, a keratinase, an esterase, a cutinase, a pullulanase, a pectinase, a mannanase, a glucosidase, a glucanase, a cholesterol oxidase, a peroxidase, and a laccase, and particularly preferred are a protease, a cellulase, an amylase, and a lipase.

Examples of the protease include commercially available Alcalase, Esperase, Everlase, Savinase, Kannase, Progress Uno (registered trademark, Novozymes A/S), PREFERENZ, EFFECTENZ, EXCELLENZ (registered trademark, DuPont de Nemours, Inc.), Lavergy (registered trademark, BASF SE), and KAP (Kao Corporation).

Examples of the cellulase include Celluclean, Carezyme (registered trademark, Novozymes A/S), KAC, and an alkaline cellulase produced by *Bacillus* sp. KSM-S237 strain described in JP-A-10-313859 and a mutant alkaline cellulase described in JP-A-2003-313592 (Kao Corporation).

Examples of the amylase include Termamyl, Duramyl, Stainzyme, Stainzyme Plus, Amplify Prime (registered trademark, Novozymes A/S), PREFERENZ, EFFECTENZ (registered trademark, DuPont de Nemours, Inc.), and KAM (Kao Corporation).

Examples of the lipase include Lipolase and Lipex (registered trademark, Novozymes A/S).

The cleansing composition can include a known detergent component, and examples of the known detergent component include the following.

### (1) Surfactant

The surfactant is blended in an amount of from 0.5 to 60 mass% in the cleansing composition, in particular, preferably in an amount of from 10 to 45 mass% in a powder cleansing composition and in an amount of from 20 to 90 mass% in a liquid cleansing composition. When the cleansing composition is a laundry clothes detergent or an automatic dishwasher detergent, the surfactant is blended generally in an amount of from 1 to 10 mass% and preferably from 1 to 5 mass%.

Examples of the surfactant that is used in the cleansing composition include, for example, one or a combination of an anionic surfactant, a nonionic surfactant other than the sulfosuccinate or a salt thereof, an amphoteric surfactant, and a cationic surfactant, and preferably an amphoteric surfactant.

Examples of the amphoteric surfactant include preferably an amine oxide surfactant or a betaine surfactant, and more preferably a tertiary amine oxide surfactant, sulfobetaine surfactant, or a carbobetaine surfactant. Examples of the tertiary amine oxide surfactant include a tertiary amine oxide surfactant in which one of the groups bound to the nitrogen atom is an alkyl group having 8 or more and 18 or less carbon atoms, preferably an alkyl group having 8 or more and 16 or less carbon atoms, further preferably an alkyl group having 8 or more and 14 or less carbon atoms, which may be interrupted by an amide group or an ester group, and the remaining group is an alkyl group having 1 or more and 3 or less carbon atoms and preferably a methyl group. Suitable examples of sulfobetaine surfactant include a compound having one alkyl group having 10 or more and 18 or less, preferably 16 or less, and more preferably 14 or less carbon atoms; two alkyl groups having 1 or more and 3 or less carbon atoms, preferably methyl groups; and a 3-sulfopropyl group or a 2-hydroxy-3-sulfopropyl group. Preferable examples of the carbobetaine surfactant include preferably a carbobetaine surfactant having one alkyl group having 10 or more and 18 or less, preferably 16 or less, and more preferably 14 or less carbon atoms, which may be interrupted by an amide group or an ester group; two alkyl groups having 1 or more and 3 or less carbon atoms, preferably methyl groups; and one carboxyalkyl group, preferably a carboxymethyl group.

### (2) Divalent metal ion scavenger

A divalent metal ion scavenger is blended in an amount of from 0.01 to 50 mass%, preferably from 5 to 40 mass%. Examples of the divalent metal ion scavenger that is used in the cleansing composition include a condensed phosphate such as a tripolyphosphate, a pyrophosphate, and an orthophosphate; an aluminosilicate such as zeolite; and a synthetic layered crystalline silicate, a nitrilotriacetate, an ethylenediaminetetraacetate, a citrate, an isocitrate, and a polyacetal carboxylate. Among these scavengers, a crystalline aluminosilicate (synthetic zeolite) is particularly preferable, and among A-type, X-type, and P-type zeolite, A-type is particularly preferable. Synthetic zeolite having an average primary particle diameter of from 0.1 to 10 µm, particularly, from 0.1 to 5 µm is suitably used.

### (3) Alkali agent

An alkali agent is blended in an amount of from 0.01 to 80 mass%, preferably from 1 to 40 mass%. Examples of the alkali agent that is used in a powder detergent include alkali metal carbonates such as sodium carbonate, collectively known as dense ash or light ash; and amorphous alkali metal silicates such as JIS Nos. 1, 2, and 3. These inorganic alkali agents are effective for particle skeleton formation during drying of the detergent and can provide a detergent that is relatively hard and has excellent fluidity. Other examples of the alkali agent include sodium sesquicarbonate and sodium hydrogen carbonate, and phosphates, such as tripolyphosphate, also have a function as an alkali agent. Examples of the alkali agent that is used in a liquid detergent include, in addition to the above-mentioned alkali agents, sodium hydroxide and mono-, di-, or tri-ethanolamine, and the alkali agent can also be used as a counterion of a surfactant.

### (4) Anti-resoiling agent

An anti-resoiling agent is blended in an amount of from 0.001 to 10 mass%, preferably from 1 to 5 mass%. Examples of the anti-resoiling agent that is used in the cleansing composition include polyethylene glycol, a carboxylic acid polymer, polyvinyl alcohol, and polyvinylpyrrolidone. Among these agents, the carboxylic acid polymer has, in addition to resoiling-preventing ability, a function of capturing metal ions and a function of dispersing solid particle soil from clothing into the wash bath. The carboxylic acid polymer is a homopolymer or a copolymer of acrylic acid, methacrylic acid, itaconic acid, or the like. A copolymer of the above-mentioned monomer and maleic acid is suitable, and the molecular weight is preferably from several thousands to 100 000. In addition to the above-mentioned carboxylic acid polymer, polymers such as polyglycidylate, cellulose derivatives such as carboxymethyl cellulose, and aminocarboxylic acid polymer such as polyaspartic acid are also preferable because of their metal ion scavenging, dispersing, and resoiling-preventing ability.

### (5) Bleaching agent

For example, a bleaching agent such as hydrogen peroxide and percarbonate is preferably blended in an amount of from 1 to 10 mass%. When a bleaching agent is used, tetraacetylethylenediamine (TAED) and a bleaching activator (activator) described in, for example, JP-A-6-316700 can be blended in an amount of from 0.01 to 10 mass%.

### (6) Fluorescent agent

Examples of a fluorescent agent that is used in the cleansing composition include a biphenyl fluorescent agent (e.g., Tinopla CBS-X) and a stilbene fluorescent agent (e.g., DM-type fluorescent dye). The fluorescent agent is preferably blended in an amount of from 0.001 to 2 mass%.

### (7) Other component

The cleansing composition can contain a solvent, a builder, a softener, a reducing agent (such as a sulfite), a foam inhibitor (such as silicone), a fragrance, an antibacterial and antifungal agent (Proxel [tradename], such as benzoic acid), and other additives that are known in the laundry detergent field.

Examples of the solvent include a monovalent alcohol having 1 or more and 3 or less carbon atoms; a multivalent alcohol having 2 or more and 4 or less carbon atoms; a di- or tri-alkylene glycol of which the alkylene glycol unit has from 2 to 4 carbon atoms; and a monoalkoxy (methoxy, ethoxy, propoxy, or butoxy), phenoxy, or benzoxy ether of di- to tetra-alkylene glycols in which the alkylene glycol unit has from 2 to 4 carbon atoms.

The solvent is preferably a water-soluble organic solvent having 2 or more, preferably 3 or more, carbon atoms and having 10 or less, preferably 8 or less, carbon atoms. Here, the water-soluble organic solvent refers to a solvent having octanol/water partition coefficient (LogPow) of 3.5 or less.

Specifically, examples of the solvent include ethanol, isopropyl alcohol; ethylene glycol, propylene glycol, glycerin, isoprene glycol; diethylene glycol, dipropylene glycol; propylene glycol monomethyl ether, propylene glycol monoethyl ether, diethylene glycol monobutyl ether (also referred to as butyl diglycol), phenoxy ethanol, phenoxy triethylene glycol, and phenoxy isopropanol. The solvent is preferably selected from the group consisting of ethanol, propylene glycol, dipropylene glycol, diethylene glycol monobutyl ether, phenoxy ethanol, phenyl glycol, and phenoxy isopropanol. The solvent preferably includes an alkoxy group and further preferably includes at least one or more selected from monoalkoxy, phenoxy, or benzoxy ether of di- to tetra-alkylene glycol in which the alkylene glycol unit has from 2 to 4 carbon atoms. The solvent more preferably contains diethylene glycol monobutyl ether.

The cleansing composition can be produced by combining the lipase mutant of the present invention obtained by the above method and the above known washing components according to a common method. The form of the detergent can be selected depending on the purpose and can be, for example, a liquid, a powder, a granule, a paste, or a solid.

The thus-obtained cleansing composition can be used as, for example, a clothes detergent, a dishwashing detergent, a bleaching agent, a detergent for washing hard surfaces, a drain pipe detergent, an artificial tooth detergent, or a bactericidal detergent for medical instruments, preferably a clothes detergent or a dishwashing detergent, and more preferably a laundry clothes detergent (laundry detergent), a dishwashing detergent for hand washing, or an automatic dishwasher detergent.

The cleansing composition is suitable for use at 40°C or less, 35°C or less, 30°C or less, or 25°C or less and at 5°C or more, 10°C or more, or 15°C or more and is suitable for use at from 5°C to 40°C, from 10°C to 35°C, from 15°C to 30°C, or from 15°C to 25°C. Examples of the preferable mode of use include use for low-temperature (from 15°C to 30°C) washing at laundries and use for low-temperature (from 15°C to 30°C) washing by automatic dishwashers.

The use of the cleansing composition of the present invention enables washing an object to be washed (for example, clothing, tableware, hard surfaces, drain pipes, artificial teeth, and medical instruments) that require the removal of stains, i.e., enables removing stains. Such a washing method includes bringing an object to be washed that requires the removal of stains into contact with the cleansing composition of the present invention. The stains are preferably oil stains.

In the washing method of the present invention, in order to bring an object to be washed and a cleansing composition into contact with each other, the object may be soaked in water in which the cleansing composition has been dissolved, or the cleansing composition may be directly applied to the object. In the method of the present invention, the object to be washed after soaking or application of a cleansing composition may be further hand washed, scrubbed with sponge, or washed with a washing machine, which is not necessarily needed.

### <6. Method for evaluating or selecting lipase>

As shown in Examples described later, the degree of alleviation of the inhibition of activity of a lipase mutant against a dispersed substrate in the presence of a surfactant strongly correlates with the improvement of the ability to remove triglycerides on a hard surface in the presence of a sulfosuccinate, compared to a parent lipase. Accordingly, a lipase having high detergency and being excellent in ability to decompose oil stains adhering to a hard surface in the presence of a sulfosuccinate or a salt thereof can be evaluated or selected by using the degree of inhibition of activity of the lipase against a dispersed substrate in the presence of a surfactant as an indicator.

Accordingly, in another aspect, the present invention provides a method for evaluating or selecting a lipase that exhibits an ability to decompose oil stains adhering to a hard surface in the presence of a sulfosuccinate or a salt thereof. The method of the present invention can be a method for evaluating or selecting a lipase having enhanced ability to decompose oil stains adhering to a hard surface in the presence of a sulfosuccinate or a salt thereof. The method of the present invention includes a step of measuring lipase activity of a test lipase against a dispersed substrate in the presence of a surfactant, a step of evaluating a degree of inhibition of lipase activity of the test lipase by the surfactant based on the measured result, and a step of evaluating or selecting a test lipase having lower degree of lipase activity inhibition by the surfactant than a standard lipase as a lipase having an ability to decompose oil stains adhering to a hard surface in the presence of a sulfosuccinate or a salt thereof.

In general, in evaluation of an enzyme for washing use, the degree of activity against a dispersed substrate and the degree of ability to remove stains adhering to a surface do not correspond to each other. In particular, when a substrate having a structure different from the substrate included in actual stains is used, they differ from each other in both the substrate structure and present state, and thus the activity measurement using the dispersed substrate is not usually valid as the evaluation of detergency. In contrast, in the present invention, it was found that the degree of activity inhibition in the presence of a surfactant can be used, instead of using specific activity of an enzyme relative to its substrate, as an indicator of detergency, and therefore the activity measurement using a dispersed substrate can be used as evaluation of detergency. The evaluation or selection method of the present invention can be carried out in a dispersion system using a dispersed substrate and is simple and high throughput. According to the evaluation or selection method of the present invention, a lipase with high detergency can be evaluated or selected rapidly with high sensitivity, compared to direct examination of ability to decompose oil stains adhering to a hard surface.

The biological species from which the test lipase used in the evaluation or selection method of the present invention is derived is not specifically limited. The test lipase may be a natural (wild-type) polypeptide or its artificial mutant. As described above, the substitution of amino acid residues at positions 120, 130, 134, 136, and 137 numbered according to SEQ ID NO: 2 is substitution for improving the detergency of the lipase, and these amino acid positions are all present in the lid domain of the lipase. Accordingly, the test lipase is preferably a mutant including at least one mutation (substitution, deletion, or insertion) in the amino acid residues of lid domain of the lipase from the viewpoint of evaluation or selection efficiency.

Examples of the surfactant include one of an anionic surfactant, a nonionic surfactant, an amphoteric surfactant, and a cationic surfactant or a combination thereof and preferably one of an anionic surfactant and a nonionic surfactant or a combination thereof, more preferably one of a sulfosuccinate or its salt, SDS, and Triton X-100 or a combination thereof, and further preferably a sulfosuccinate or its salt. The sulfosuccinate or a salt thereof is the same as those described in detail as a component that can be included in the cleansing composition containing the lipase mutant of the present invention.

In the evaluation or selection method of the present invention, the lipase activity of a test lipase against a dispersed substrate in the presence of a surfactant is measured. The lipase activity of a test lipase against a dispersed substrate in the presence of a surfactant can be measured using a well-known method in the art. The lipase activity value against a dispersed substrate in the presence of a surfactant can be determined by, for example, mixing a test lipase and a surfactant solution, adding a solution of 4-nitrophenyl octanoate as a dispersed substrate to the resulting mixture solution, measuring the absorbance change (OD/min) at 405 nm due to release of 4-nitrophenol, and determining the difference ΔOD/min from a blank (sample without addition of a lipase).

Subsequently, in the evaluation or selection method of the present invention, the degree of inhibition of lipase activity of the test lipase by the surfactant is evaluated based on the measured result. The degree of inhibition of lipase activity of a test lipase against a dispersed substrate in the presence of a surfactant can be estimated by, for example, comparing the lipase activity of a test lipase against a dispersed substrate in the presence of a surfactant and the lipase activity of a standard lipase against a dispersed substrate in the presence of the surfactant. Specifically, the higher the lipase activity of a test lipase against a dispersed substrate in the presence of a surfactant compared to the lipase activity of a standard lipase against a dispersed substrate in the presence of the surfactant, the smaller the degree of inhibition of lipase activity of the test lipase against a dispersed substrate in the presence of a surfactant can be evaluated to be, and the lower the lipase activity of a test lipase against a dispersed substrate in the presence of a surfactant compared to the lipase activity of a standard lipase against a dispersed substrate in the presence of the surfactant, the larger the degree of inhibition of lipase activity of the test lipase against a dispersed substrate in the presence of a surfactant can be evaluated to be. Here, as the standard lipase, a known lipase can be used. Various lipases are known from literature, and a lipase consisting of the amino acid sequence of SEQ ID NO: 2, 4, 6, or 8 is suitably used as the standard lipase. Alternatively, when a mutant is used as the test lipase, a parent lipase of the test lipase is preferably used as the standard lipase. Alternatively, a model lipase established so as to have predetermined lipase activity against a dispersed substrate in the presence of a surfactant can be used. The lipase activity of the model lipase against a dispersed substrate in the presence of a surfactant may be appropriately set based on the performance of the lipase to be evaluated or selected.

Alternatively, in the evaluation of the degree of inhibition of lipase activity of a test lipase by a surfactant, the lipase activity of the test lipase against a dispersed substrate in the absence of a surfactant is determined as in the measurement of the lipase activity of the test lipase against a dispersed substrate in the presence of a surfactant except that a solution not containing a surfactant is used instead of the surfactant solution, and the lipase activity of the test lipase against a dispersed substrate in the presence of a surfactant may be compared to the lipase activity against the dispersed substrate in the absence of a surfactant. For example, the ΔOD/min ratio is determined by dividing the lipase activity represented by ΔOD/min of a test lipase in the presence of a surfactant by the lipase activity value represented by ΔOD/min of the test lipase in the absence of a surfactant. The ΔOD/min ratio is a relative measure of the lipase activity retention rate in the presence of a surfactant compared to in the absence of a surfactant, and a smaller value thereof shows that the lipase activity is inhibited in the presence of a surfactant. Accordingly, using such a value as an indicator, the degree of lipase activity inhibition by a surfactant can be evaluated. Accordingly, the evaluation or selection method of the present invention may further include a step of measuring the lipase activity of a test lipase against a dispersed substrate in the absence of a surfactant.

Subsequently, in the evaluation or selection method of the present invention, a test lipase which exhibits lower degree of lipase activity inhibition by the surfactant than a standard lipase is evaluated or selected as a lipase having an ability to decompose oil stains adhering to hard surfaces in the presence of a sulfosuccinate or a salt thereof.

The evaluation or selection of a lipase having an ability to decompose oil stains adhering to a hard surface in the presence of a sulfosuccinate or a salt thereof in the present invention is performed by comparing the degree of inhibition of lipase activity of a test lipase by a surfactant to the degree of inhibition of lipase activity of a standard lipase by the surfactant. For example, when the degree of inhibition of lipase activity of a test lipase by a surfactant is statistically significantly smaller than the degree of inhibition of lipase activity of a standard lipase by the surfactant, the test lipase can be evaluated or selected as a lipase having an ability to decompose oil stains adhering to a hard surface in the presence of a surfactant. In addition, when the degree of inhibition of lipase activity of a test lipase by a surfactant is smaller than the degree of inhibition of lipase activity of a standard lipase by the surfactant, for example, by preferably 10% or more, more preferably 20% or more, and further preferably 30% or more, the test lipase can be evaluated or selected as a lipase having an ability to decompose oil stains adhering to a hard surface in the presence of a surfactant. In case of where the degree of inhibition of lipase activity of a lipase by a surfactant is evaluated by the ΔOD/min ratio, when the ΔOD/min ratio of a test lipase is higher, for example, significantly higher, or higher by preferably 10% or more, more preferably 20% or more, further preferably 30% or more, or further preferably 300% or more than the ΔOD/min ratio of a standard lipase under the same conditions, the test lipase can be evaluated or selected as a lipase having an ability to decompose oil stains adhering to a hard surface in the presence of a surfactant.

An example of a specific procedure of the evaluation or selection method of the present invention will now be shown, but the method is not limited to this method.

4-Nitrophenyl octanoate (SIGMA) is used as a substrate. A model washing solution described in Table 3 below or 20 mM Tris-HCl (pH 7.0) is used as the test solution, 4-nitrophenyl octanoate is added to each test solution at a final concentration of 2 mM, and each of the resulting mixtures is used as a substrate solution. Two microliter of a test lipase appropriately diluted with 20 mM Tris-HCl (pH 7.0) and 100 µL of the substrate solution were mixed in each well of a 96-well assay plate, and the absorbance change (OD/min) at 405 nm is measured at 30°C. The difference ΔOD/min between the OD/min in each test solution and a blank (sample without addition of a lipase) is determined as the lipase activity in the presence of a surfactant and the lipase activity in the absence of a surfactant. Subsequently, the ΔOD/min ratio that is an indicator of the degree of inhibition of lipase activity against a dispersed substrate in the presence of a surfactant is determined by dividing the lipase activity represented by ΔOD/min in the presence of a surfactant by the lipase activity represented by ΔOD/min in the absence of a surfactant. Subsequently, the ΔOD/min ratio of the test lipase is compared to the ΔOD/min ratio of a standard lipase determined by the same way, and a test lipase having higher the ΔOD/min ratio than the ΔOD/min ratio of the standard lipase is evaluated or selected as a lipase having an ability to decompose oil stains adhering to a hard surface in the presence of a surfactant.

The thus-obtained lipase exhibits an ability to decompose oil stains adhering to a hard surface in the presence of a surfactant, has excellent detergency, and is useful as an enzyme that is blended in various cleansing compositions. The details of the composition, the form, the mode of use, and so on of the cleansing composition are the same as those of the cleansing composition including the lipase mutant of the present invention.

Regarding the above-described embodiments, in the present invention, the following aspects are further disclosed.
<1> A lipase mutant that consists of an amino acid sequence having an identity of at least 75% with the amino acid sequence of SEQ ID NO: 2, 4, 6, or 8 and has one or more, preferably two or more, more preferably three or more, further preferably four or more, and further preferably all amino acid residues selected from the group consisting of the following (a) to (e):
(a) an amino acid residue other than L at a position corresponding to position 120 numbered according to SEQ ID NO: 2;
(b) an amino acid residue other than S at a position corresponding to position 130 numbered according to SEQ ID NO: 2;
(c) an amino acid residue other than A at a position corresponding to position 134 numbered according to SEQ ID NO: 2;
(d) an amino acid residue other than L at a position corresponding to position 136 numbered according to SEQ ID NO: 2; and
(e) an amino acid residue other than S at a position corresponding to position 137 numbered according to SEQ ID NO: 2,
(provided that, when the lipase mutant has only an amino acid residue of (d) in the above (a) to (e) and the amino acid residue is M, the lipase mutant consists of an amino acid sequence having an identity of at least 93% with the amino acid sequence of SEQ ID NO: 2, 4, or 6).
<2> The lipase mutant according to <1>, wherein the (a) to (e) are the following (a') to (e'), respectively:
(a') A, F, G, H, I, K, M, N, Q, R, S, T, V, W, or Y at a position corresponding to position 120 numbered according to SEQ ID NO: 2;
(b') A, C, E, F, G, H, I, K, L, M, Q, R, T, V, W, or Y at a position corresponding to position 130 numbered according to SEQ ID NO: 2;
(c') C, D, E, G, I, T, or V at a position corresponding to position 134 numbered according to SEQ ID NO: 2;
(d') A, C, E, F, G, H, I, M, S, T, V, or W at a position corresponding to position 136 numbered according to SEQ ID NO: 2; and
(e') C, N, or T at a position corresponding to position 137 numbered according to SEQ ID NO: 2,
(provided that, when the lipase mutant has only M of (d') in the above (a') to (e'), the lipase mutant consists of an amino acid sequence having an identity of at least 93% with the amino acid sequence of SEQ ID NO: 2, 4, or 6).
<3> The lipase mutant according to <2>, in which the lipase mutant has two or more amino acid residues selected from the group consisting of the (a') to (e').
<4> The lipase mutant according to any one of <1> to <3>, in which the lipase mutant has any of amino acid residues Nos. 1 to 75 in Table 2 below.

**[Table 2]**

| No. | Amino acid residue |
|---|---|
| 1 | 120A |
| 2 | 120F |
| 3 | 120G |
| 4 | 120H |
| 5 | 120I |
| 6 | 120K |
| 7 | 120M |
| 8 | 120N |
| 9 | 120Q |
| 10 | 120R |
| 11 | 120S |
| 12 | 120T |
| 13 | 120V |
| 14 | 120W |
| 15 | 120Y |
| 16 | 130A |
| 17 | 130C |
| 18 | 130E |
| 19 | 130F |
| 20 | 130H |
| 21 | 130I |
| 22 | 130K |
| 23 | 130L |
| 24 | 130M |
| 25 | 130Q |
| 26 | 130R |
| 27 | 130T |
| 28 | 130V |
| 29 | 130W |
| 30 | 130Y |
| 31 | 134C |
| 32 | 134D |
| 33 | 134E |
| 34 | 134G |
| 35 | 134I |
| 36 | 134T |
| 37 | 134V |
| 38 | 136A |
| 39 | 136C |
| 40 | 136E |
| 41 | 136F |
| 42 | 136G |
| 43 | 136H |
| 44 | 136I |
| 45 | 136M |
| 46 | 136T |
| 47 | 136V |
| 48 | 136W |
| 49 | 137C |
| 50 | 137N |
| 51 | 137T |
| 52 | 120G 130A |
| 53 | 120G 130L |
| 54 | 120H 130A |
| 55 | 120H 130L |
| 56 | 120R 130A |
| 57 | 120R 130K |
| 58 | 120R 130L |
| 59 | 120S 130A |
| 60 | 120S 130K |
| 61 | 120S 130L |
| 62 | 120S 134T |
| 63 | 120S 136A |
| 64 | 120S 136S |
| 65 | 120S 137N |
| 66 | 130A 134T |
| 67 | 130A 136M |
| 68 | 130A 137T |
| 69 | 130K 136M |
| 70 | 130M 137T |
| 71 | 130Y 137T |
| 72 | 136M 137N |
| 73 | 120S 130A 136M |
| 74 | 120R 130K 136M 137N |
| 75 | 120R 130A 134T 135V 136M 137N |

| | |
|---|---|
| *The amino acid position is a position numbered according to SEQ ID NO: 2. | |

<5> The lipase mutant according to <4>, wherein the lipase mutant is a lipase mutant consisting of an amino acid sequence having an identity of at least 75% with the amino acid sequence of SEQ ID NO: 2 and having any of amino acid residues Nos. 1 to 74 in the above Table 2, a lipase mutant consisting of an amino acid sequence having an identity of at least 75% with the amino acid sequence of SEQ ID NO: 4 and having any of amino acid residues Nos. 11, 59, and 75 in the above Table 2, a lipase mutant consisting of an amino acid sequence having an identity of at least 75% with the amino acid sequence of SEQ ID NO: 6 and having the amino acid residue No. 59 in the above Table 2, or a lipase mutant consisting of an amino acid sequence having an identity of at least 75% with the amino acid sequence of SEQ ID NO: 8 and having any of amino acid residues Nos. 4, 6, 11, 22, 24, 30, 36, 41, 43, 50, 51, and 59 in the above Table 2.
<6> A polynucleotide encoding the lipase mutant according to any one of <1> to <5>.
<7> A vector or DNA fragment comprising the polynucleotide according to <6>.
<8> A transformed cell comprising the vector or DNA fragment according to <7>.
<9> The transformed cell according to <8>, wherein the transformed cell is a microorganism.
<10> The transformed cell according to <9>, wherein the transformed cell is *Escherichia coli* or a *Bacillus* bacterium, preferably a *Bacillus* bacterium, and more preferably *Bacillus subtilis.*
<11> A method for producing a lipase mutant, comprising a step of culturing the transformed cell according to any one of <8> to <10>.
<12> A cleansing composition comprising the lipase mutant according to any one of <1> to <5>.
<13> The cleansing composition according to <12>, further comprising a sulfosuccinate or a salt thereof, preferably a branched alkyl sulfosuccinate having a branched-chain alkyl group having 9 or more and 12 or less carbon atoms or a salt thereof, more preferably a branched alkyl sulfosuccinate having a branched-chain alkyl group having 9 or 10 carbon atoms or a salt thereof, and further preferably a branched alkyl sulfosuccinate having a branched-chain alkyl group having 10 carbon atoms or a salt thereof.
<14> The cleansing composition according to <12>, further comprising a diester sulfosuccinate or a salt thereof, preferably a branched dialkyl sulfosuccinate in which two branched-chain alkyl groups are each a branched-chain alkyl group having 9 or more and 12 or less carbon atoms or a salt thereof, more preferably a branched dialkyl sulfosuccinate in which two branched-chain alkyl groups are each a branched-chain alkyl group having 9 or 10 carbon atoms or a salt thereof, further preferably a branched dialkyl sulfosuccinate in which two branched-chain alkyl groups are each a branched-chain alkyl group having 10 carbon atoms or a salt thereof, and further preferably bis-(2-propylheptyl) sulfosuccinate or a salt thereof.
<15> The cleansing composition according to any one of <12> to <14>, wherein the cleansing composition is a clothes detergent or a dishwashing detergent.
<16> The cleansing composition according to any one of <12> to <15>, wherein the cleansing composition is a powder or a liquid.
<17> The cleansing composition according to any one of <12> to <16>, wherein the cleansing composition is used at low temperature.
<18> The cleansing composition according to <17>, wherein the cleansing composition is used at 40°C or less, 35°C or less, 30°C or less, or 25°C or less and 5°C or more, 10°C or more, or 15°C or more, or is used at from 5°C to 40°C, from 10°C to 35°C, from 15°C to 30°C, or from 15°C to 25°C.
<19> A stain-washing method comprising using the cleansing composition according to any one of <12> to <18>.
<20> The method according to <19>, comprising bringing an object to be washed into contact with the cleansing composition according to any one of <12> to <18>.
<21> Use of the lipase mutant according to any one of <1> to <5> for producing a cleansing composition.
<22> The use of the lipase mutant according to any one of <1> to <5> for washing a stain.
<23> A method for producing a lipase mutant, comprising performing one or more steps, preferably two or more, more preferably three or more, and further preferably four or more and further preferably all steps selected from the group consisting of the following (i) to (v) in a polypeptide that consists of an amino acid sequence having an identity of at least 75% with the amino acid sequence of SEQ ID NO: 2, 4, 6, or 8 and has lipase activity:
(i) a step of substituting the amino acid residue at a position corresponding to position 120 numbered according to SEQ ID NO: 2 with an amino acid residue other than L;
(ii) a step of substituting the amino acid residue at a position corresponding to position 130 numbered according to SEQ ID NO: 2 with an amino acid residue other than S;
(iii) a step of substituting the amino acid residue at a position corresponding to position 134 numbered according to SEQ ID NO: 2 with an amino acid residue other than A;
(iv) a step of substituting the amino acid residue at a position corresponding to position 136 numbered according to SEQ ID NO: 2 with an amino acid residue other than L; and
(v) a step of substituting the amino acid residue at a position corresponding to position 137 numbered according to SEQ ID NO: 2 with an amino acid residue other than S, (provided that, when the method comprises performing only the step (iv) in the above (i) to (v) and the amino acid residue is substituted with M, the polypeptide consists of an amino acid sequence having an identity of at least 93% with the amino acid sequence of SEQ ID NO: 2, 4, or 6).

<24> The method according to <23>, wherein the (i) to (v) are the following (i') to (v'), respectively:
(i') a step of substituting the amino acid residue at a position corresponding to position 120 numbered according to SEQ ID NO: 2 with A, F, G, H, I, K, M, N, Q, R, S, T, V, W, or Y;
(ii') a step of substituting the amino acid residue at a position corresponding to position 130 numbered according to SEQ ID NO: 2 with A, C, E, F, G, H, I, K, L, M, Q, R, T, V, W, or Y;
(iii') a step of substituting the amino acid residue at a position corresponding to position 134 numbered according to SEQ ID NO: 2 with C, D, E, G, I, T, or V;
(iv') a step of substituting the amino acid residue at a position corresponding to position 136 numbered according to SEQ ID NO: 2 with A, C, E, F, G, H, I, M, S, T, V, or W; and
(v') a step of substituting the amino acid residue at a position corresponding to position 137 numbered according to SEQ ID NO: 2 with C, N, or T, (provided that, when the method comprises performing only the step (iv') in the above (i') to (v') and the amino acid residue is substituted with M, the polypeptide consists of an amino acid sequence having an identity of at least 93% with the amino acid sequence of SEQ ID NO: 2, 4, or 6).

<25> The method according to <24>, wherein two or more steps selected from the group consisting of the (i') to (v') are performed.
<26> The method according to any one of <23> to <25>, wherein the substitution is substitution with any of amino acid residues Nos. 1 to 75 in the above Table 2.
<27> The method according to <26>, wherein the substitution is substitution with any of amino acid residues Nos. 1 to 74 of the above Table 2 in a polypeptide that consists of an amino acid sequence having an identity of at least 75% with the amino acid sequence of SEQ ID NO: 2 and has lipase activity, substitution with any of amino acid residues Nos. 11, 59, and 75 in the above Table 2 in a polypeptide that consists of an amino acid sequence having an identity of at least 75% with the amino acid sequence of SEQ ID NO: 4 and has lipase activity, substitution with the amino acid residue No. 59 in the above Table 2 in a polypeptide that consists of an amino acid sequence having an identity of at least 75% with the amino acid sequence of SEQ ID NO: 6 and has lipase activity, or substitution with any of amino acid residues Nos. 4, 6, 11, 22, 24, 30, 36, 41, 43, 50, 51, and 59 in the above Table 2 in a polypeptide that consists of an amino acid sequence having an identity of at least 75% with the amino acid sequence of SEQ ID NO: 8 or has lipase activity.
<28> A method for evaluating or selecting a lipase having an ability to decompose an oil stain adhering to a hard surface in the presence of a sulfosuccinate or a salt thereof, comprising steps of:
measuring lipase activity of a test lipase against a dispersed substrate in the presence of a surfactant;
evaluating a degree of inhibition of lipase activity of the test lipase by the surfactant based on the measured result; and
evaluating or selecting a test lipase having a lower degree of lipase activity inhibition by the surfactant than a standard lipase as a lipase having an ability to decompose an oil stain adhering to a hard surface in the presence of a sulfosuccinate or a salt thereof.

<29> The method according to <28>, further comprising a step of measuring the lipase activity of the test lipase against a dispersed substrate in the absence of a surfactant.
<30> The method according to <28> or <29>, wherein the surfactant further comprises a sulfosuccinate or a salt thereof, preferably a branched alkyl sulfosuccinate having a branched-chain alkyl group having 9 or more and 12 or less carbon atoms or a salt thereof, more preferably a branched alkyl sulfosuccinate having a branched-chain alkyl group having 9 or 10 carbon atoms or a salt thereof, and further preferably a branched alkyl sulfosuccinate having a branched-chain alkyl group having 10 carbon atoms or a salt thereof.
<31> The method according to <28> or <29>, wherein the surfactant further comprises s a diester sulfosuccinate or a salt thereof, preferably a branched dialkyl sulfosuccinate in which two branched-chain alkyl groups are each a branched-chain alkyl group having 9 or more and 12 or less carbon atoms or a salt thereof, more preferably a branched dialkyl sulfosuccinate in which two branched-chain alkyl groups are each a branched-chain alkyl group having 9 or 10 carbon atoms or a salt thereof, further preferably a branched dialkyl sulfosuccinate in which two branched-chain alkyl groups are each a branched-chain alkyl group having 10 carbon atoms or a salt thereof, and further preferably bis-(2-propylheptyl) sulfosuccinate or a salt thereof.
<32> The method according to any one of <28> to <31>, wherein the test lipase is a lipase mutant comprising mutation of at least one amino acid residue in the lid domain of a lipase.

### Examples

The present invention will be described in further detail based on Examples below, but is not limited thereto.

### (1) Construction of lipase expression plasmid

Lipase expression plasmids below were constructed by using a plasmid for expressing VHH of SEQ ID NO: 26 including a promoter derived from a *Bacillus subtilis* spoVG gene described in WO 2021/153129 as a template and transferring each lipase genes to a full-length ORF including a VHH gene by In-Fusion reaction. Plasmids pHY-CnLip, pHY-EbLip, pHY-Ag1Lip, pHY-Lipr138, and pHY-Lipr139 were constructed respectively from artificially synthesized lipase genes CnLip, EbLip, Ag1Lip, Liprl38, and Lipr139 (respectively polynucleotides of SEQ ID NOs: 1, 3, 5, 9, and 11 encoding the amino acid sequences of SEQ ID NOs: 2, 4, 6, 10, and 12, respectively).

A plasmid pHY-amyEsig-TLL was constructed by transferring, to the full-length ORF including the Lipr139 gene of plasmid pHY-Lipr139, an artificially synthesized lipase TLL gene (the polynucleotide of SEQ ID NO: 13 encoding the amino acid sequence of SEQ ID NO: 14) in which a signal sequence (the polynucleotide of SEQ ID NO: 15 encoding the amino acid sequence of SEQ ID NO: 16) derived from *Bacillus subtilis* amyE was linked to the N-terminus by In-Fusion reaction.

A plasmid pHY-CspLip was constructed by using the plasmid pHY-S237 described in Example 7 of WO 2006/068148 as a template and transferring artificially synthesized CspLip gene (the polynucleotide of SEQ ID NO: 7 encoding the amino acid sequence of SEQ ID NO: 8) to the full-length ORF of an alkaline cellulase gene with by In-Fusion reaction.

In the mutagenesis on each lipase, site-directed mutagenesis by PCR using a complementary primer pair (Zheng, Lei, Ulrich Baumann, and Jean-Louis Reymond, Nucleic Acids Research, 32, 14 (2004): ell5-ell5.) was used.

### (2) Preparation of lipase solution

A lipase expression plasmid was introduced into a *Bacillus subtilis* strain by a protoplast method. The strain was cultured in 2 × L-maltose culture medium (2%tryptone, 1% yeast extract, 1% NaCl, 7.5% maltose, 7.5 ppm manganese sulfate pentahydrate, 0.04% calcium chloride dihydrate, and 15 ppm tetracycline: % is (w/v)%) at 30°C for 3 days, and the culture supernatant containing the lipase was then collected by centrifugation.

The culture supernatant and 2 × Laemmli Sample buffer (Bio-Rad) containing 100 mM DTT were mixed at equal volumes, and the mixture was incubated at 100°C for 5 minutes. Six microliter of each sample was applied to each lane of Any kDTM Mini-PROTEAN (registered trademark) TGX Stain-Free TM Protein Gel (Bio-Rad) and was then subjected to electrophoresis at a constant voltage of 200 V. The gel after the electrophoresis was photographed using Chemi Doc MP Imaging system (Bio-Rad), and the band intensity of a band that is inferred as a lipase was quantitatively measured. A calibration curve was produced from band intensity of BSA, and the concentration of lipase in each culture supernatant was calculated.

### (3) Measurement of activity against dispersed substrate in model washing solution

4-Nitrophenyl octanoate (SIGMA) was used as a substrate. The activity of lipase can be determined by measuring the rate of increase in absorbance due to release of 4-nitrophenol by the function of a lipase. Model washing solutions (Table 3) and 20 mM Tris-HCl (pH 7.0) were used as test solutions, and 4-nitrophenyl octanoate was added to and mixed with each test solution at a final concentration of 2 mM to prepare each substrate solution. Two microliter of the culture supernatant containing a lipase diluted 200 times with 20 mM Tris-HCl (pH 7.0) and 100 µL of each substrate solution were mixed in each well of a 96-well assay plate, and the absorbance change (OD/min) at 405 nm was measured at 30°C. The difference ΔOD/min from a blank (sample without addition of an enzyme) was determined.

The degree of the alleviation of a mutant was calculated as follows. For each lipase, the value (ΔOD/min ratio) was calculated by dividing the ΔOD/min when a model washing solution was used as a test solution by the ΔOD/min when 20 mM Tris-HCl (pH 7.0) was used as a test solution. The ΔOD/min ratio is a relative measure of the activity retention rate in a model washing solution compared to in a buffer, and a smaller value thereof shows that the activity is inhibited in the model washing solution. The value obtained by dividing the ΔOD/min ratio of the mutant by the ΔOD/min ratio of the wild-type lipase thereof was determined as the degree of the alleviation of a mutant. When the degree of the alleviation is larger than 1, the activity inhibition in the model washing solution is alleviated compared to the wild-type lipase.

The relative activity (%) was calculated as follows. Two microliter of 20 mM Tris-HCl (pH 7.0) including from 47 to 1500 µM 4-nitrophenol was mixed with 100 µL of a model washing solution or 20 mM Tris-HCl (pH 7.0), the absorbance of each of the resulting mixtures at 405 nm was measured to produce a calibration curve. The release rate of 4-nitrophenol per minute (µM/min) was calculated from the calibration curve and the value of ΔOD/min of each test solution. For each lipase, the value obtained by dividing the release rate (µM/min) when the model washing solution was used as a test solution by the release rate (µM/min) when the 20 mM Tris-HCl (pH 7.0) was used as a test solution and multiplying the resulting quotient by 100 was determined as the relative activity (%).

**[Table 3]**

| Mixture component | mass% |
|---|---|
| Branched alkyl sulfosuccinate | 0.2 |
| Betaine | 2 |
| Butyl diglycol | 6.5 |
| Water | Balance |
| Total | 100 |

| | |
|---|---|
| • Branched alkyl sulfosuccinate : sodium bis-(2-propylheptyl)sulfosuccinate • Betaine : lauryl(2-hydroxy-3-sulfopropyl)dimethyl betaine, AMPHITOL 20HD, manufactured by Kao Corporation | |

### (4) Measurement of activity against dispersed substrate in surfactant solution

A solution prepared by adding 20 mM 4-nitrophenyl octanoate to 20 mM Tris-HCl (pH 7.0) was used as the substrate solution. A solution prepared by adding Triton X-100 to 20 mM Tris-HCl (pH 7.0) at 0.1% (w/v) was used as a surfactant solution. Two microliter of the culture supernatant containing a lipase diluted 200 times with 20 mM Tris-HCl (pH 7.0) and 100 µL of a surfactant solution were mixed in each well of a 96-well assay plate, 10 µL of the substrate solution was added thereto, and the absorbance change (OD/min) at 405 nm was measured at 30°C. The difference ΔOD/min from a blank (sample without addition of an enzyme) was defined as the active value. For each lipase, the value obtained by dividing the activity value in the surfactant solution by the activity value (the activity in the buffer) when 20 mM Tris-HCl (pH 7.0) was used instead of the surfactant solution and multiplying the resulting quotient by 100 was determined as the relative activity (%).

### (5) Evaluation of washing in model washing solution

The ability to remove triglycerides on a hard surface was evaluated as detergency. Beef tallow (SIGMA, 03-0660) and rapeseed oil (SIGMA, 23-0450) were mixed at a weight ratio of 9 : 1, the mixture was dissolved in triple volumes of chloroform, and the solution was then colored with 0.2 wt% Sudan III to give a model stain. Ten microliter of the model stain was dropwise added to each well bottom of a 96-well polypropylene deep well plate, and the chloroform was evaporated and dried to make a stain plate.

A washing solution was prepared by adding a lipase solution to the model washing solution described in Table 3. Soak washing was performed by gently adding 300 µL of the washing solution to each well of the stain plate and leaving it to stand at room temperature (about 22°C) for from 10 to 30 minutes. One hundred microliter of each washing solution was taken from each well while avoiding contact with the stain on the bottom and was transferred to another 9-well plate. In order to quantitatively measure Sudan III in a model stain that was solubilized in the washing solution by the soak washing, the absorbance (A500) at 500 nm was measured. A500 corresponds to the release amount of oil into the washing solution and can be used as an indicator of detergency. The enzymatic effect (ΔA500) on detergency was determined by subtracting A500 of a washing solution containing 20 mM Tris-HCl (pH 7.0) instead of the lipase from A500 of the washing solution containing each lipase.

### (6) Measurement of activity of wild-type lipase in model washing solution

The relative activity of each wild-type lipase in the model washing solution relative to that in a buffer was measured by the method described in (3). All of Lipr138 whose suitability for washing was disclosed in Patent Literature 2, Lipr139 whose suitability for washing was disclosed in Patent Literature 1, and CnLip had significantly inhibited activity against a dispersed substrate in a model washing solution (Table 4).

**[Table 4]**

| Lipase | Relative activity % |
|---|---|
| Lipr138 | 0 |
| Lipr139 | 2.8 |
| CnLip | 4.6 |

### (7) Measurement of activity of CnLip mutant in model washing solution

The relative activity of each mutant of CnLip in the model washing solution relative to that in a buffer was measured by the method described in (3). The activity inhibition in the model washing solution was significantly alleviated by substitution of position 120, 130, 134, 136, or 137 (Table 5).

**[Table 5]**

| Lipase | Degree of alleviation |
|---|---|
| Wild-type CnLip (parent enzyme) | 1 |
| L120A | 1.89 |
| L120F | 2.53 |
| L120G | 3.46 |
| L120H | 4.58 |
| L120I | 2.99 |
| L120K | 3.07 |
| L120M | 3.02 |
| L120N | 1.81 |
| L120Q | 2.22 |
| L120R | 3.62 |
| L120S | 2.18 |
| L120T | 2.95 |
| L120V | 3.1 |
| L120W | 5.16 |
| L120Y | 4.93 |
| S130A | 3.98 |
| S130C | 3.93 |
| S130E | 2.71 |
| S130F | 5.77 |
| S130H | 1.85 |
| S130I | 5.34 |
| S130K | 2.61 |
| S130L | 3.72 |
| S130M | 5.21 |
| S130Q | 1.93 |
| S130R | 4.07 |
| S130T | 2.68 |
| S130V | 2.17 |
| S130W | 1.97 |
| S130Y | 3.65 |
| A134C | 2.94 |
| A134D | 2.23 |
| A134E | 1.56 |
| A134G | 2.01 |
| A134I | 1.93 |
| A134T | 1.34 |
| A134V | 1.65 |
| L136A | 2.1 |
| L136C | 3.03 |
| L136E | 4.33 |
| L136F | 6.61 |
| L136G | 2.88 |
| L136H | 5.57 |
| L136I | 1.49 |
| L136M | 3.13 |
| L136T | 1.63 |
| L136V | 1.34 |
| L136W | 4.85 |
| S137C | 1.38 |
| S137N | 2.31 |
| S137T | 2.13 |

### (8) Measurement of activity of CnLip multiple mutant in model washing solution

For multiple mutants of CnLip including substitutions of two or more residues at positions selected from the group consisting of positions 120, 130, 134, 136, and 137, the relative activity in the model washing solution relative to that in a buffer was measured by the method described in (3). The activity inhibition in the model washing solution was further alleviated by combining substitutions of two or more residues (Table 6).

**[Table 6]**

| Lipase | Degree of alleviation |
|---|---|
| Wild-type CnLip (parent enzyme) | 1 |
| L120S S130A | 4.7 |
| L120S A134T | 3.63 |
| L120SS137N | 4.19 |
| L120S S130K | 5.11 |
| L120S S130L | 4.88 |
| L120S L136A | 4.8 |
| L120H S130A | 7.91 |
| L120G S130A | 6.32 |
| L120R S130A | 5.16 |
| L120R S130K | 4.19 |
| L120H S130L | 5 |
| L120R S130L | 7.77 |
| L120G S130L | 5.65 |
| S130A A134T | 4 |
| S130A L136M | 5.22 |
| L136M S137N | 4.52 |
| S130K L136M | 4.63 |
| L120S S130A L136M | 5.85 |
| L120S L136S | 3.67 |
| S130A S137T | 4.16 |
| S130M S137T | 5.47 |
| S130Y S137T | 7.13 |
| L120R S130K L136M S137N | 6.47 |

### (9) Measurement of activity of CnLip mutant in surfactant solution

The activity of CnLip mutants in the presence of Triton X-100 was measured by the method described in (4). For the mutants which had decreased activity inhibition in the model washing solution, the activity inhibition in the presence of Triton-100 was also decreased (Table 7).

**[Table 7]**

| Lipase | Relative activity % |
|---|---|
| Wild-type CnLip (parent enzyme) | 13.29 |
| L120S | 17.36 |
| L120S S130A | 21.5 |
| L120H | 24.84 |
| L120K | 17.71 |
| L120G | 22.54 |
| S130M | 25.63 |
| S130Y | 21.45 |
| L120S S137N | 24.6 |
| L120S S130K | 26.95 |
| L120S S130L | 19.46 |
| L136M | 19.37 |
| S137N | 22.27 |
| L120R S130A | 27.33 |
| L120R S130K | 25.78 |
| L136M S137N | 25.04 |

### (10) Evaluation of detergency of CnLip mutant

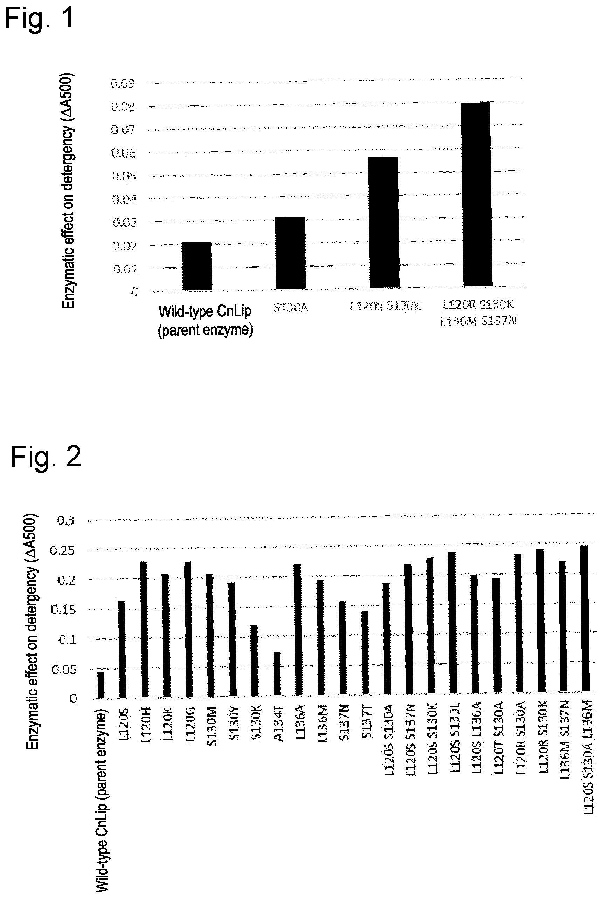

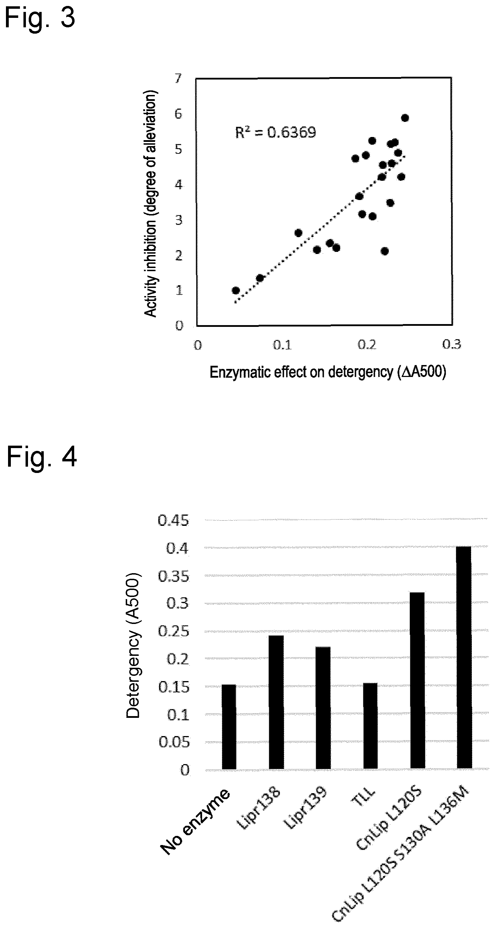

The detergency of each of the wild-type lipase and CnLip mutants in the model washing solution was evaluated by the method described in (5). The culture supernatant was diluted with 20 mM Tris-HCl (pH 7.0) to a lipase concentration of 200 mg/L, the resulting lipase solution was added in an amount of 1/50 of the model washing solution, and the resulting solution mixture was subjected to soak washing for 10 minutes or 30 minutes. The results of washing for 10 minutes are shown in Figure 1, and the results of washing for 30 minutes are shown in Figure 2. In the mutants which had decreased inhibition of the activity against the dispersed substrate in the model washing solution or in the surfactant solution, the performance of removing triglycerides on a hard surface in the model washing solution was notably improved. When the degree of the alleviation of activity inhibition measured in (7) and (8) and the enzymatic effect on detergency in washing for 30 minutes were plotted, a strong correlation was observed (Figure 3). It was demonstrated from the above results that a lipase having improved performance of removing triglycerides on a hard surface in a washing solution containing an alkyl sulfosuccinate can be easily screened by measuring the activity against a dispersed substrate or the degree of activity inhibition in the model washing solution or the surfactant solution.

The detergency of CnLip mutant was compared to those of Lipr138, Lipr139, and TLL by a similar test, and as a result, the CnLip mutant with improved detergency exhibited significantly high detergency compared to Lipr138, Lipr139, and TLL (Figure 4).

### (11) Measurement of activity of EbLip, AglLip, and CspLip mutants in model washing solution

The relative activity of each mutant of EbLip, AglLip, and CspLip in the model washing solution relative to that in a buffer was measured by the method described in (3) (Table 8). As in CnLip, the activity inhibition in the model washing solution was notably alleviated by substitution of one or more residues at positions selected from the group consisting of positions 120, 130, 134, 136, and 137 for EbLip and Ag1Lip or substitution of one or more residues at positions selected from the group consisting of positions 121, 131, 135, 137, and 138 for CspLip (corresponding to positions 120, 130, 134, 136, and 137 of CnLip, respectively).

**[Table 8]**

| Parent enzyme | Mutation | Degree of alleviation |
|---|---|---|
| EbLip | None | 1 |
| | L120S | 2.2 |
| | L120S S130A | 4.62 |
| | L120S S130A A134T A135V L136M S137N | 8.6 |
| Ag1Lip | None | 1 |
| | L120S S130A | 1.3 |
| | None | 1 |
| | L121S | 1.16 |
| | L121H | 1.43 |
| | L121K | 1.2 |
| | S131M | 2.21 |
| | S131Y | 2.47 |
| CspLip | S131K | 1.26 |
| | A135T | 1.23 |
| | M137F | 1.69 |
| | M137H | 1.65 |
| | S138N | 1.79 |
| | S138T | 2.27 |
| | L121S S131A | 1.62 |

### (12) Evaluation of detergency of CspLip mutant

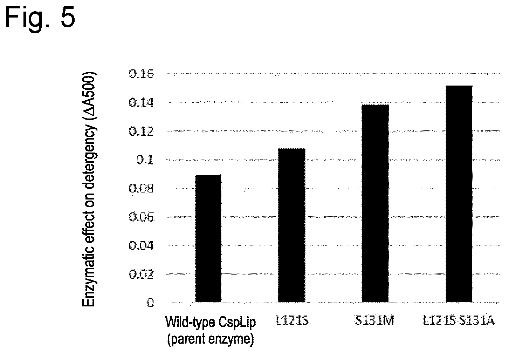

The detergency of each mutant of CspLip in the model washing solution was evaluated by the method described in (5). The culture supernatant was diluted to a lipase concentration of 200 mg/L, the resulting lipase solution was added in an amount of 1/50 of the model washing solution, and the mixture was subjected to soak washing for 30 minutes. The results are shown in Figure 5. As in CnLip, in the mutants which had decreased inhibition of the activity against the dispersed substrate in the model washing solution or the surfactant solution, the performance of removing triglycerides on a hard surface in the model washing solution was notably improved.

## Claims

1. A lipase mutant that consists of an amino acid sequence having an identity of at least 75% with the amino acid sequence of SEQ ID NO: 2, 4, 6, or 8 and has one or more amino acid residues selected from the group consisting of the following (a) to (e):
(a) an amino acid residue other than leucine at a position corresponding to position 120 numbered according to SEQ ID NO: 2;
(b) an amino acid residue other than serine at a position corresponding to position 130 numbered according to SEQ ID NO: 2;
(c) an amino acid residue other than alanine at a position corresponding to position 134 numbered according to SEQ ID NO: 2;
(d) an amino acid residue other than leucine at a position corresponding to position 136 numbered according to SEQ ID NO: 2; and
(e) an amino acid residue other than serine at a position corresponding to position 137 numbered according to SEQ ID NO: 2,
(provided that, when the lipase mutant has only an amino acid residue of (d) in the above (a) to (e) and the amino acid residue is methionine, the lipase mutant consists of an amino acid sequence having an identity of at least 93% with the amino acid sequence of SEQ ID NO: 2, 4, or 6).

2. The lipase mutant according to claim 1, wherein the (a) to (e) are the following (a') to (e'), respectively:
(a') alanine, phenylalanine, glycine, histidine, isoleucine, lysine, methionine, asparagine, glutamine, arginine, serine, threonine, valine, tryptophan, or tyrosine at a position corresponding to position 120 numbered according to SEQ ID NO: 2;
(b') alanine, cysteine, glutamic acid, phenylalanine, glycine, histidine, isoleucine, lysine, leucine, methionine, glutamine, arginine, threonine, valine, tryptophan, or tyrosine at a position corresponding to position 130 numbered according to SEQ ID NO: 2;
(c') cysteine, aspartic acid, glutamic acid, glycine, isoleucine, threonine, or valine at a position corresponding to position 134 numbered according to SEQ ID NO: 2;
(d') alanine, cysteine, glutamic acid, phenylalanine, glycine, histidine, isoleucine, methionine, serine, threonine, valine, or tryptophan at a position corresponding to position 136 numbered according to SEQ ID NO: 2; and
(e') cysteine, asparagine, or threonine at a position corresponding to position 137 numbered according to SEQ ID NO: 2,
(provided that, when the lipase mutant has only methionine of (d') in the above (a') to (e'), the lipase mutant consists of an amino acid sequence having an identity of at least 93% with the amino acid sequence of SEQ ID NO: 2, 4, or 6).

3. The lipase mutant according to claim 2, wherein the lipase mutant has two or more amino acid residues selected from the group consisting of the (a') to (e').

4. A polynucleotide encoding the lipase mutant according to any one of claims 1 to 3.

5. A vector or DNA fragment comprising the polynucleotide according to claim 4.

6. A transformed cell comprising the vector or DNA fragment according to claim 5.

7. The transformed cell according to claim 6, wherein the transformed cell is a microorganism.

8. A cleansing composition comprising the lipase mutant according to any one of claims 1 to 3.

9. The cleansing composition according to claim 8, wherein the cleansing composition is a clothes detergent or a dishwashing detergent.

10. A stain-washing method comprising using the cleansing composition according to claim 8 or 9.

11. A method for producing a lipid mutant, comprising performing one or more steps selected from the group consisting of the following (i) to (v) in a polypeptide that consists of an amino acid sequence having an identity of at least 75% with the amino acid sequence of SEQ ID NO: 2, 4, 6, or 8 and has a lipase activity:
(i) a step of substituting an amino acid residue at a position corresponding to position 120 numbered according to SEQ ID NO: 2 with an amino acid residue other than leucine;
(ii) a step of substituting an amino acid residue at a position corresponding to position 130 numbered according to SEQ ID NO: 2 with an amino acid residue other than serine;
(iii) a step of substituting an amino acid residue at a position corresponding to position 134 numbered according to SEQ ID NO: 2 with an amino acid residue other than alanine;
(iv) a step of substituting an amino acid residue at a position corresponding to position 136 numbered according to SEQ ID NO: 2 with an amino acid residue other than leucine; and
(v) a step of substituting an amino acid residue at a position corresponding to position 137 numbered according to SEQ ID NO: 2 with an amino acid residue other than serine,
(provided that, when the method comprises performing only the step (iv) in the above (i) to (v) and the amino acid residue is substituted with methionine, the polypeptide consists of an amino acid sequence having an identity of at least 93% with the amino acid sequence of SEQ ID NO: 2, 4, or 6).

12. A method for evaluating or selecting a lipase having an ability to decompose an oil stain adhering to a hard surface in the presence of a sulfosuccinate or a salt thereof, comprising steps of:
measuring lipase activity of a test lipase against a dispersed substrate in the presence of a surfactant;
evaluating a degree of inhibition of lipase activity of the test lipase by the surfactant based on the measured result; and
evaluating or selecting a test lipase which has lower degree of lipase activity inhibition by the surfactant than a standard lipase as a lipase having an ability to decompose an oil stain adhering to a hard surface in the presence of a sulfosuccinate or a salt thereof.
